# EUROPEAN PATENT SPECIFICATION

(11) **EP 2 478 916 B1**
(45) Date of publication and mention of the grant of the patent: **01.04.2020**
(21) Application number: 11184022.9
(22) Date of filing: 26.01.2007
(51) Int. Cl.: A61K 39/145

(54) **Influenza vaccines containing hemagglutinin and matrix proteins**
Influenzaimpfstoffe mit Hämagglutinin und Matrixproteinen
Vaccins contre la grippe contenant de l'hémagglutinine et des protéines de matrice

(30) Priority: 27.01.2006 GB 0601733; 11.10.2006 GB 0620175
(43) Date of publication of application: 25.07.2012
(62) Divisional of application: 07734467.9
(73) Proprietor: Seqirus UK Limited, Maidenhead, Berkshire SL6 8AA (GB)
(72) Inventor: Broeker, Michael, 35041 Marburg (DE); Kost, Holger, 35041 Marburg (DE)
(74) Representative: Wise, Daniel Joseph

(56) References cited:
- EP-A1- 0 870 508
- US-A- 5 741 493
- CHALOUPKA I ET AL: "Comparative analysis of six European influenza vaccines", EUROPEAN JOURNAL OF CLINICAL MICROBIOLOGY & INFECTIOUS DISEASES, SPRINGER, WIESBADEN, DE, vol. 15, no. 2, 1 January 1996 (1996-01-01), pages 121-127, XP009101927, ISSN: 0934-9723, DOI: 10.1007/BF01591484
- WONG S S Y ET AL: "Influenza vaccination: options and issues", HONG KONG MEDICAL JOURNAL, vol. 11, no. 5, October 2005 (2005-10), pages 381-390, XP002677577, ISSN: 1024-2708
- HOCART M ET AL: "Preparation and characterization of a purified influenza virus neuraminidase vaccine", VACCINE, ELSEVIER LTD, GB, vol. 13, no. 18, 1 January 1995 (1995-01-01), pages 1793-1798, XP004057386, ISSN: 0264-410X, DOI: 10.1016/0264-410X(95)00126-L
- PLOTNICKY H ET AL: "The immunodominant influenza matrix T cell epitope recognized in human induces influenza protection in HLA-A2/Kb transgenic mice.", VIROLOGY, vol. 309, no. 2, 10 May 2003 (2003-05-10), pages 320-329, XP002449311, ISSN: 0042-6822
- GARCIA-CANAS ET AL: "Rapid and selective characterization of influenza virus constituents in monovalent and multivalent preparations using non-porous reversed-phase high performance liquid chromatography columns", JOURNAL OF CHROMATOGRAPHY, ELSEVIER SCIENCE PUBLISHERS B.V. AMSTERDAM, NL, vol. 1123, no. 2, 11 August 2006 (2006-08-11), pages 225-232, XP005585075, ISSN: 0021-9673
- Julian Hickling MBA, PhD and Erik D'Hondt PhD: "A review of production technologies for influenza virus vaccines, and their suitability for deployment in developing countries for influenza pandemic preparedness", World Health OrganizationInitiative for Vaccine Research , 20 December 2006 (2006-12-20), pages 1-34, Retrieved from the Internet: URL:http://who.int/vaccine_research/diseas es/influenza/Flu_vacc_manuf_tech_report.pd f [retrieved on 2013-01-16]
- BRANDS R ET AL: "Influvac: a safe Madin Darby Canine Kidney (MDCK) cell culture-based influenza vaccine.", DEVELOPMENTS IN BIOLOGICAL STANDARDIZATION 1999, vol. 98, 1999, pages 93-100 ; dis, ISSN: 0301-5149
- JOHANSSON B E ET AL: "Influenza: A unique problem in vaccination", FUTURE VIROLOGY 2010 FUTURE MEDICINE LTD. GBR, vol. 5, no. 5, September 2010 (2010-09), pages 651-664, ISSN: 1746-0794
- JOHANSSON BERT E ET AL: "Immunization with dissociated neuraminidase, matrix, and nucleoproteins from influenza A virus eliminates cognate help and antigenic competition", VIROLOGY,, vol. 225, no. 1, 1 November 1996 (1996-11-01), pages 136-144, XP002560702, ISSN: 0042-6822 [retrieved on 2002-04-22]
- CROWE ET AL: "Identification of protective and non-protective T cell epitopes in influenza", VACCINE, ELSEVIER LTD, GB, vol. 24, no. 4, 23 January 2006 (2006-01-23), pages 452-456, XP005225984, ISSN: 0264-410X, DOI: 10.1016/J.VACCINE.2005.07.090
- MERTEN O W ET AL: "PRODUCTION OF INFLUENZA VIRUS IN CELL CULTURES FOR VACCINE PREPARATION", ADVANCES IN EXPERIMENTAL MEDICINE AND BIOLOGY, SPRINGER, US, vol. 397, 1 January 1996 (1996-01-01), pages 141-151, XP000982880, ISSN: 0065-2598

## Description

### TECHNICAL FIELD

This invention is in the field of vaccines for protecting against influenza virus infection, and in particular vaccines that include matrix proteins, as defined in the claims.

### BACKGROUND ART

Various forms of influenza virus vaccine are currently available (*e.g.* see chapters 17 & 18 of reference 1). Vaccines are generally based either on live virus or on inactivated virus. Inactivated vaccines may be based on whole virions, 'split' virions, or on purified surface antigens.

Haemagglutinin (HA) is the main immunogen in inactivated influenza vaccines, and vaccine doses are standardized by reference to HA levels, typically as measured by a single radial immunodiffusion (SRID) assay. Current vaccines typically contain about 15µg of HA per strain per dose. In addition to containing influenza HA, vaccines can include further influenza virus proteins. For instance, all current vaccines include neuraminidase (NA). Reference 2 reports that European influenza vaccines can also include significant amounts of other influenza virus proteins. For example, matrix (M) protein was found in split vaccines but not in purified surface antigen vaccines.

In addition to the influenza virus antigens, reference 2 reports that vaccines also contain egg-derived proteins, such as ovalbumin. These proteins arise because the standard method for influenza virus growth in vaccine manufacture uses embryonated hen eggs, with virus being purified from the egg contents (allantoic fluid).

Chaloupka et al. (European Journal of Clinical Microbiology and Infectious Disease (1996) 15(2): 121-127), Wong et al. (Hong Kong Medical Journal (2005) 11(5): 381-390), and US 5,741,493A discuss vaccines prepared from influenza viruses cultured on eggs. EP0870508A1 relates to methods in which influenza virus is grown in mammalian cell culture. Hocart et al. (Vaccine (1995) 13(18): 1793-1798) discusses the preparation and characterization of a purified influenza virus neuraminidase vaccine.

### DISCLOSURE OF THE INVENTION

Rather than use eggs for viral growth in vaccine manufacture, it has been proposed to grow viruses in cell culture. While investigating this technique, the inventors unexpectedly detected matrix sequences. In particular, whereas reference 2 found no matrix protein in purified surface antigen vaccines prepared from virions grown on eggs, the inventors detected matrix protein in purified surface antigen vaccines prepared from virions grown in cell culture.

The invention provides a method for preparing an immunogenic composition comprising the steps of: (i) growing influenza virus in cell culture wherein trypsin is added to allow viral release; (ii) preparing an antigen composition from the viruses grown in step (i), wherein the antigen composition comprises haemagglutinin and matrix proteins not as a whole virion which form a stable complex; and (iii) combining the antigen composition with a pharmaceutical carrier, to give the immunogenic composition, wherein the matrix protein has an amino acid sequence which is a fragment of a full-length M1 matrix protein amino acid sequence.

The immunogenic composition comprises influenza virus haemagglutinin and matrix proteins, prepared from virus grown in cell culture.

The disclosure includes an immunogenic composition comprising influenza virus haemagglutinin and matrix proteins, wherein the composition does not contain ovalbumin. The composition can also be free from other egg proteins (*e.g.* ovomucoid) and from chicken DNA.

The disclosure includes a method for preparing an immunogenic composition comprising the steps of: (i) growing influenza virus in cell culture; (ii) preparing an antigen composition from the viruses grown in step (i), wherein the antigen composition comprises haemagglutinin and matrix proteins; and (iii) combining the antigen composition with a pharmaceutical carrier, to give the immunogenic composition.

A particular matrix protein that has been seen is a fragment of M1. Full-length M1 protein is a 27.8kDa protein, but the observed fragment has a molecular weight of about 5kDa on a low MW SDS-PAGE gel (see below). It includes a highly-conserved amino acid sequence LSYSXGALA (SEQ ID NO: 1), where X is A or T. Thus the disclosure includes an immunogenic composition comprising influenza virus haemagglutinin and matrix proteins, wherein: the matrix protein has a molecular weight of less than 20kDa and comprises an amino acid sequence having at least 50% identity (*e.g.* at least 60%, 70%, 80%, 85%, 90%, 95% or more) to SEQ ID NO: 1. Another M1 fragment that has been observed is around 75aa long and lacks the N-terminal methionine of the full M1 sequence (*e.g.* it has amino acid sequence SEQ ID NO: 28 or SEQ ID NO: 29). Thus the disclosure includes an immunogenic composition comprising influenza virus haemagglutinin and matrix proteins, wherein the matrix protein is a M1 protein lacking the N-terminal methionine of the natural M1 sequence. The matrix protein may comprise an amino acid sequence having at least 50% identity (*e.g.* at least 60%, 70%, 80%, 85%, 90%, 95% or more) to SEQ ID NO: 28 or to SEQ ID NO: 29. In addition to lacking the N-terminal methionine, the fragment may lack one or more further amino acids downstream from the N-terminal methionine.

Although these compositions are preferably prepared from virus grown in cell culture, they can alternatively be prepared in other ways *e.g.* by adding the matrix protein to an egg-derived vaccine, by using a purification protocol with egg-derived virions which results in the production and presence of the matrix protein, by combining the matrix protein with a recombinantly-expressed HA (and, optionally, other recombinantly-expressed proteins), *etc.*

### Preparation of antigen components

The invention does not use a whole virion (WV) antigen *i.e.* it does not encompass vaccines that use a live virus or a whole inactivated virion. Instead, the antigens of the invention are non-WV antigens, such as split virions, or purified surface antigens. Compositions of the disclosure comprise at least two influenza virus antigens: haemagglutinin and matrix. They may also include other influenza virus antigen(s), such as neuraminidase. The antigens will typically be prepared from influenza virions (grown in cell culture) but, in some embodiments, the antigens can be expressed in a recombinant host (*e.g.* in an insect cell line using a baculovirus vector) and used in purified form [3,4]. In general, however, antigens will be from virions.

In preparing non-WV antigens from virions, the virions may be inactivated. Chemical means for inactivating a virus include treatment with an effective amount of one or more of the following agents: detergents, formaldehyde, β-propiolactone, methylene blue, psoralen, carboxyfullerene (C60), binary ethylamine, acetyl ethyleneimine, or combinations thereof. Non-chemical methods of viral inactivation are known in the art, such as for example UV light or gamma irradiation.

Virions can be harvested from virus-containing fluids by various methods. For example, a purification process may involve zonal centrifugation using a linear sucrose gradient solution that includes detergent to disrupt the virions. Antigens may then be purified, after optional dilution, by diafiltration.

Split virions are obtained by treating purified virions with detergents (*e.g.* ethyl ether, polysorbate 80, deoxycholate, tri-*N*-butyl phosphate, Triton X-100, Triton N101, cetyltrimethylammonium bromide, Tergitol NP9, *etc*.) to produce subvirion preparations, including the 'Tween-ether' splitting process. Methods of splitting influenza viruses are well known in the art *e.g.* see refs. 5-10, *etc.* Splitting of the virus is typically carried out by disrupting or fragmenting whole virus, whether infectious or non-infectious with a disrupting concentration of a splitting agent. The disruption results in a full or partial solubilisation of the virus proteins, altering the integrity of the virus. Preferred splitting agents are non-ionic and ionic (*e.g.* cationic) surfactants *e.g.* alkylglycosides, alkylthioglycosides, acyl sugars, sulphobetaines, betains, polyoxyethylenealkylethers, N,N-dialkyl-Glucamides, Hecameg, alkylphenoxy-polyethoxyethanols, quaternary ammonium compounds, sarcosyl, CTABs (cetyl trimethyl ammonium bromides), tri-*N*-butyl phosphate, Cetavlon, myristyltrimethylammonium salts, lipofectin, lipofectamine, and DOT-MA, the octyl- or nonylphenoxy polyoxyethanols (*e.g.* the Triton surfactants, such as Triton X-100 or Triton N101), polyoxyethylene sorbitan esters (the Tween surfactants), polyoxyethylene ethers, polyoxyethlene esters, *etc.* One useful splitting procedure uses the consecutive effects of sodium deoxycholate and formaldehyde, and splitting can take place during initial virion purification (e.g. in a sucrose density gradient solution). Thus a splitting process can involve clarification of the virion-containing material (to remove non-virion material), concentration of the harvested virions (*e.g.* using an adsorption method, such as CaHPO₄ adsorption), separation of whole virions from non-virion material, splitting of virions using a splitting agent in a density gradient centrifugation step (*e.g.* using a sucrose gradient that contains a splitting agent such as sodium deoxycholate), and then filtration (*e.g.* ultrafiltration) to remove undesired materials. Split virions can usefully be resuspended in sodium phosphate-buffered isotonic sodium chloride solution. The BEGRIVAC™, FLUARIX™, FLUZONE™ and FLUSHIELD™ products are split vaccines.

Purified surface antigen vaccines comprise the influenza surface antigens haemagglutinin and, typically, also neuraminidase. Processes for preparing these proteins in purified form are well known in the art. The FLUVIRIN™, AGRIPPAL™ and INFLUVAC™ products are subunit vaccines.

Influenza antigens can also be presented in the form of virosomes [11] (nucleic acid free viral-like liposomal particles), as in the INFLEXAL V™ and INVAVAC™ products, but it is preferred not to use virosomes with the present invention. Thus, in some embodiments, the influenza antigen is not in the form of a virosome.

The influenza virus may be attenuated. The influenza virus may be temperature-sensitive. The influenza virus may be cold-adapted. These three features are particularly useful when using live virus as an antigen.

Influenza virus strains for use in vaccines change from season to season. In the current inter-pandemic period, vaccines typically include two influenza A strains (H1N1 and H3N2) and one influenza B strain, and trivalent vaccines are typical. The invention may also use HA from pandemic strains (*i.e*. strains to which the vaccine recipient and the general human population are immunologically naive), such as H2, H5, H7 or H9 subtype strains (in particular of influenza A virus), and influenza vaccines for pandemic strains may be monovalent or may be based on a normal trivalent vaccine supplemented by a pandemic strain. Depending on the season and on the nature of the antigen included in the vaccine, however, it may protect against one or more of influenza A virus HA subtypes H1, H2, H3, H4, H5, H6, H7, H8, H9, H10, H11, H12, H13, H14, H15 or H16. The vaccine may protect against one or more of influenza A virus NA subtypes N1, N2, N3, N4, N5, N6, N7, N8 or N9.

As well as being suitable for immunizing against inter-pandemic strains, the compositions of the disclosure are particularly useful for immunizing against pandemic strains. The characteristics of an influenza strain that give it the potential to cause a pandemic outbreak are: (a) it contains a new hemagglutinin compared to the hemagglutinins in currently-circulating human strains, *i.e*. one that has not been evident in the human population for over a decade (*e.g.* H2), or has not previously been seen at all in the human population (*e.g*. H5, H6 or H9, that have generally been found only in bird populations), such that the human population will be immunologically naive to the strain's hemagglutinin; (b) it is capable of being transmitted horizontally in the human population; and (c) it is pathogenic to humans. A virus with H5 haemagglutinin type is preferred for immunizing against pandemic influenza, such as a H5N1 strain. Other possible strains include H5N3, H9N2, H2N2, H7N1 and H7N7, and any other emerging potentially pandemic strains. Within the H5 subtype, a virus may fall into HA clade 1, HA clade 1', HA clade 2 or HA clade 3 [12], with clades 1 and 3 being particularly relevant.

Other strains whose antigens can usefully be included in the compositions are strains which are resistant to antiviral therapy (*e.g.* resistant to oseltamivir [13] and/or zanamivir), including resistant pandemic strains [14].

Compositions of the disclosure may include antigen(s) from one or more (*e.g.* 1, 2, 3, 4 or more) influenza virus strains, including influenza A virus and/or influenza B virus. Monovalent vaccines are not preferred, and where a vaccine includes more than one strain of influenza, the different strains are typically grown separately and are mixed after the viruses have been harvested and antigens have been prepared. Thus a process of the invention may include the step of mixing antigens from more than one influenza strain. A trivalent vaccine is preferred, including antigens from two influenza A virus strains and one influenza B virus strain.

In some embodiments of the invention, the compositions may include antigen from a single influenza A strain. In some embodiments, the compositions may include antigen from two influenza A strains, provided that these two strains are not H1N1 and H3N2. In some embodiments, the compositions may include antigen from more than two influenza A strains.

The influenza virus may be a reassortant strain, and may have been obtained by reverse genetics techniques. Reverse genetics techniques [*e.g.* 15-19] allow influenza viruses with desired genome segments to be prepared *in vitro* using plasmids. Typically, it involves expressing (a) DNA molecules that encode desired viral RNA molecules *e.g.* from poll promoters, and (b) DNA molecules that encode viral proteins *e.g.* from polII promoters, such that expression of both types of DNA in a cell leads to assembly of a complete intact infectious virion. The DNA preferably provides all of the viral RNA and proteins, but it is also possible to use a helper virus to provide some of the RNA and proteins. Plasmid-based methods using separate plasmids for producing each viral RNA are preferred [20-22], and these methods will also involve the use of plasmids to express all or some (*e.g.* just the PB1, PB2, PA and NP proteins) of the viral proteins, with 12 plasmids being used in some methods.

To reduce the number of plasmids needed, a recent approach [23] combines a plurality of RNA polymerase I transcription cassettes (for viral RNA synthesis) on the same plasmid (*e.g.* sequences encoding 1, 2, 3, 4, 5, 6, 7 or all 8 influenza A vRNA segments), and a plurality of protein-coding regions with RNA polymerase II promoters on another plasmid (*e.g.* sequences encoding 1, 2, 3, 4, 5, 6, 7 or all 8 influenza A mRNA transcripts). Preferred aspects of the reference 23 method involve: (a) PB1, PB2 and PA mRNA-encoding regions on a single plasmid; and (b) all 8 vRNA-encoding segments on a single plasmid. Including the NA and HA segments on one plasmid and the six other segments on another plasmid can also facilitate matters.

As an alternative to using poll promoters to encode the viral RNA segments, it is possible to use bacteriophage polymerase promoters [24]. For instance, promoters for the SP6, T3 or T7 polymerases can conveniently be used. Because of the species-specificity of poll promoters, bacteriophage polymerase promoters can be more convenient for many cell types (*e.g.* MDCK), although a cell must also be transfected with a plasmid encoding the exogenous polymerase enzyme.

In other techniques it is possible to use dual poll and polII promoters to simultaneously code for the viral RNAs and for expressible mRNAs from a single template [25,26].

Thus an influenza A virus may include one or more RNA segments from a A/PR/8/34 virus (typically 6 segments from A/PR/8/34, with the HA and N segments being from a vaccine strain, *i.e.* a 6:2 reassortant). It may also include one or more RNA segments from a A/WSN/33 virus, or from any other virus strain useful for generating reassortant viruses for vaccine preparation. Typically, the it protects against a strain that is capable of human-to-human transmission, and so the strain's genome will usually include at least one RNA segment that originated in a mammalian (*e.g.* in a human) influenza virus. It may include NS segment that originated in an avian influenza virus.

The viruses used as the source of the antigens are generally grown on cell culture but, in some aspects of the disclosure, they may be grown on eggs. The current standard method for influenza virus growth uses specific pathogen-free (SPF) embryonated hen eggs, with virus being purified from the egg contents (allantoic fluid). More recently, however, viruses have been grown in animal cell culture and, for reasons of speed and patient allergies, this growth method is preferred. If egg-based viral growth is used then one or more amino acids may be introduced into the allantoic fluid of the egg together with the virus [10].

The cell substrate will typically be a cell line of mammalian origin. Suitable mammalian cells of origin include, but are not limited to, hamster, cattle, primate (including humans and monkeys) and dog cells. Various cell types may be used, such as kidney cells, fibroblasts, retinal cells, lung cells, *etc.* Examples of suitable hamster cells are the cell lines having the names BHK21 or HKCC. Suitable monkey cells are *e.g.* African green monkey cells, such as kidney cells as in the Vero cell line. Suitable dog cells are *e.g.* kidney cells, as in the MDCK cell line. Thus suitable cell lines include, but are not limited to: MDCK; CHO; 293T; BHK; Vero; MRC-5; PER.C6; WI-38; *etc.* The use of mammalian cells means that vaccines can be free from chicken DNA, as well as being free from egg proteins (such as ovalbumin and ovomucoid), thereby reducing allergenicity. Preferred mammalian cell lines for growing influenza viruses include: MDCK cells [27-30], derived from Madin Darby canine kidney; Vero cells [31-33], derived from African green monkey (*Cercopithecus aethiops*) kidney; or PER.C6 cells [34], derived from human embryonic retinoblasts. These cell lines are widely available *e.g.* from the American Type Cell Culture (ATCC) collection [35], from the Coriell Cell Repositories [36], or from the European Collection of Cell Cultures (ECACC). For example, the ATCC supplies various different Vero cells under catalog numbers CCL-81, CCL-81.2, CRL-1586 and CRL-1587, and it supplies MDCK cells under catalog number CCL-34. PER.C6 is available from the ECACC under deposit number 96022940. As a less-preferred alternative to mammalian cell lines, virus can be grown on avian cell lines [*e.g.* refs. 37-39], including cell lines derived from ducks (*e.g.* duck retina) or hens. Examples of avian cell lines include avian embryonic stem cells [37,40] and duck retina cells [38]. Suitable avian embryonic stem cells, include the EBx cell line derived from chicken embryonic stem cells, EB45, EB14, and EB14-074 [41]. Chicken embryo fibroblasts (CEF) may also be used.

The most preferred cell lines for growing influenza viruses are MDCK cell lines. The original MDCK cell line is available from the ATCC as CCL-34, but derivatives of this cell line may also be used. For instance, reference 27 discloses a MDCK cell line that was adapted for growth in suspension culture ('MDCK 33016', deposited as DSM ACC 2219). Similarly, reference 42 discloses a MDCK-derived cell line that grows in suspension in serum-free culture ('B-702', deposited as FERM BP-7449). Reference 43 discloses non-tumorigenic MDCK cells, including 'MDCK-S' (ATCC PTA-6500), 'MDCK-SF101' (ATCC PTA-6501), 'MDCK-SF102' (ATCC PTA-6502) and 'MDCK-SF103' (PTA-6503). Reference 44 discloses MDCK cell lines with high susceptibility to infection, including 'MDCK.5F1' cells (ATCC CRL-12042). Any of these MDCK cell lines can be used.

The culture for cell growth, and also the viral inoculum used to start the culture, will preferably be free from (*i.e.* will have been tested for and given a negative result for contamination by) herpes simplex virus, respiratory syncytial virus, parainfluenza virus 3, SARS coronavirus, adenovirus, rhinovirus, reoviruses, polyomaviruses, birnaviruses, circoviruses, and/or parvoviruses [45]. Absence of herpes simplex viruses is particularly preferred.

Virus may be grown on cells in suspension [46] or in adherent culture. In one embodiment, the cells may be adapted for growth in suspension. One suitable MDCK cell line that is adapted for growth in suspension culture is MDCK 33016 (deposited as DSM ACC 2219). As an alternative, microcarrier culture can be used.

Cell lines supporting influenza virus replication are preferably grown in serum-free culture media and/or protein free media. A medium is referred to as a serum-free medium in the context of the present invention in which there are no additives from serum of human or animal origin. Protein-free is understood to mean cultures in which multiplication of the cells occurs with exclusion of proteins, growth factors, other protein additives and non-serum proteins, but can optionally include proteins such as trypsin or other proteases that may be necessary for viral growth. The cells growing in such cultures naturally contain proteins themselves.

Cell lines supporting influenza virus replication are preferably grown below 37°C [47] (*e.g.* 30-36°C) during viral replication.

The method for propagating virus in cultured cells generally includes the steps of inoculating the cultured cells with the strain to be cultured, cultivating the infected cells for a desired time period for virus propagation, such as for example as determined by virus titer or antigen expression (*e.g.* between 24 and 168 hours after inoculation) and collecting the propagated virus. The cultured cells are inoculated with a virus (measured by PFU or TCID₅₀) to cell ratio of 1:500 to 1:1, preferably 1:100 to 1:5, more preferably 1:50 to 1:10. The virus is added to a suspension of the cells or is applied to a monolayer of the cells, and the virus is absorbed on the cells for at least 60 minutes but usually less than 300 minutes, preferably between 90 and 240 minutes at 25°C to 40°C, preferably 28°C to 37°C. The infected cell culture (*e.g.* monolayers) may be removed either by freeze-thawing or by enzymatic action to increase the viral content of the harvested culture supernatants. The harvested fluids are then either inactivated or stored frozen. Cultured cells may be infected at a multiplicity of infection ("m.o.i.") of about 0.0001 to 10, preferably 0.002 to 5, more preferably to 0.001 to 2. Still more preferably, the cells are infected at a m.o.i of about 0.01. Infected cells may be harvested 30 to 60 hours post infection. Preferably, the cells are harvested 34 to 48 hours post infection. Still more preferably, the cells are harvested 38 to 40 hours post infection. Trypsin is added during cell culture to allow viral release, and may be added at any suitable stage during the culture.

HA is the main immunogen in current inactivated influenza vaccines, and vaccine doses are standardised by reference to HA levels, typically measured by SRID. Existing vaccines typically contain about 15µg of HA per strain, although lower doses can be used *e.g.* for children, or in pandemic situations, or when using an adjuvant. Fractional doses such as ½ (*i.e.* 7.5µg HA per strain), ¼ and ⅛ have been used [89,90], as have higher doses (*e.g.* 3x or 9x doses [48,49]). Thus vaccines may include between 0.1 and 150µg of HA per influenza strain, preferably between 0.1 and 50µg *e.g.* 0.1-20µg, 0.1-15µg, 0.1-10µg, 0.1-7.5µg, 0.5-5µg, *etc.* Particular doses include *e.g.* about 45, about 30, about 15, about 10, about 7.5, about 5, about 3.8, about 1.9, about 1.5, *etc.* per strain.

For live vaccines, dosing is measured by median tissue culture infectious dose (TCID₅₀) rather than HA content, and a TCID₅₀ of between 10⁶ and 10⁸ (preferably between 10^{6.5}-10^{7.5}) per strain is typical.

HA used with the invention may be a natural HA as found in a virus, or may have been modified. For instance, it is known to modify HA to remove determinants (*e.g.* hyper-basic regions around the cleavage site between HA1 and HA2) that cause a virus to be highly pathogenic in avian species, as these determinants can otherwise prevent a virus from being grown in eggs.

### The matrix protein

As well as including haemagglutinin, compositions of the disclosure include a matrix protein. Segment 7 of the influenza A virus encodes the M1 and M2 polypeptides. M1 underlies the viral lipid bilayer, whereas M2 is an integral membrane protein that provides an ion channel which is inhibited by amantadine. M2 is expressed from a spliced mRNA. Segment 7 of influenza B virus encodes the M1 and BM2 polypeptides.

The matrix protein included in compositions of the disclosure is typically a M1 protein from an influenza A virus. The full-length 252aa M1 sequence from the PR/8/34 influenza A virus is available in the databases under GI:138817, which is SEQ ID NO: 2 herein:

The matrix protein included in compositions of the disclosure preferably comprise a M1 amino acid sequence that is at least *m* amino acids long, where said *m* amino acids have at least *n%* identity to SEQ ID NO: 2. The *m* amino acids will typically include a fragment of at least *p* consecutive amino acids from SEQ ID NO: 2.

The value of *m* can be, for example, 7, 8, 9, 10, 12, 14, 16, 18, 20, 25, 30, 35, 40, 45, 50, 60, 70, 80, 90, 100 or more. The value of *n* can be, for example, 70 (*e.g.* 75, 80, 85, 90, 91, 92, 93, 94, 95, 96, 97, 98, 99 or more). The value ofp can be, for example, 5, 6, 7, 8, 9, 10, 12, 14, 16, 18, 20, 25, 30, 35, 40, 45, 50, 60, 70, 80, 90, 100 or more. Where *n*<100, *p* will be less than *m.*

The sequence of *m* amino acids may, compared to SEQ ID NO: 2, include one or more (*e.g.* 1, 2, 3, 4, 5, 6, 7, 8, 9, 10, *etc*.) conservative amino acid replacements *i.e.* replacements of one amino acid with another which has a related side chain. Genetically-encoded amino acids are generally divided into four families: (1) acidic *i.e.* aspartate, glutamate; (2) basic *i.e.* lysine, arginine, histidine; (3) nonpolar *i.e.* alanine, valine, leucine, isoleucine, proline, phenylalanine, methionine, tryptophan; and (4) uncharged polar *i.e.* glycine, asparagine, glutamine, cysteine, serine, threonine, tyrosine. Phenylalanine, tryptophan, and tyrosine are sometimes classified jointly as aromatic amino acids. In general, substitution of single amino acids within these families does not have a major effect on the biological activity. The *m* amino acids may also include one or more (*e.g.* 1, 2, 3, 4, 5, 6, 7, 8, 9, 10, *etc.*) single amino acid deletions relative to SEQ ID NO: 2. The *m* amino acids may also include one or more (*e.g.* 1, 2, 3, 4, 5, 6, 7, 8, 9, 10, *etc.*) insertions (*e.g.* each of 1, 2, 3, 4 or 5 amino acids) relative to SEQ ID NO: 2.

Preferred matrix proteins for inclusion in compositions of the disclosure comprise an epitope from M1. The epitope may be a T-cell and/or a B-cell epitope. T- and B-cell epitopes can be identified empirically (*e.g.* using PEPSCAN [50,51] or similar methods), or they can be predicted (*e.g.* using the Jameson-Wolf antigenic index [52], matrix-based approaches [53], TEPITOPE [54], neural networks [55], OptiMer & EpiMer [56, 57], ADEPT [58], Tsites [59], hydrophilicity [60], antigenic index [61] or the methods disclosed in reference 62 *etc.*)*.* By such methods, T-cell epitopes have already been identified in influenza A virus M1 protein, including the following [63]:

| **MHC** | **Sequence** | **SEQ ID** | **REF** |
|---|---|---|---|
| HLA-A*0201 | GILGFVFTL | 3 | 64 |
| HLA-A*0201 | ILGFVFTLTV | 4 | 64 |
| HLA-A*1101 | SIIPSGPLK | 5 | 65 |
| H-2K^{b} | MGLIYNRM | 6 | 66 |
| HLA-B*3501 | ASCMGLIY | 7 | 67 |
| HLA-CW*0102 | ILSPLTKGI | 8 | 68 |
| HLA-CW*0102 | ILSPLTKGIL | 9 | 68 |
| HLA-DQw1 | AYQKRMGVQMQR | 10 | 69 |
| HLA-DQw3 | LENLQAYQKR | 11 | 69 |
| HLA-DRB1*0101 | GPLKAEIAQRLE | 12 | 70 |
| Saoe-G*02 | RKLKREITF | 13 | 71 |
| Saoe-G*04 | RKLKREITFH | 14 | 71 |

Other T cell epitopes in M1 include: SLLTEVETYV (SEQ ID NO: 15; residues 2-11 of SEQ ID NO: 2); IIPSGPLK (SEQ ID NO: 16; residues 14-21 of SEQ ID NO: 2); and LEDVFAGK (SEQ ID NO: 17; residues 28-35 of SEQ ID NO: 2).

A particular matrix protein that was first detected in vaccines prepared in cell culture is a 5kDa protein with N-terminal sequence EISLSYSAGALA (SEQ ID NO: 18; residues 114-125 of SEQ ID NO: 2). The N-terminal residue and size of this polypeptide are consistent with it being a tryptic fragment of M1, in which case the full-length polypeptide might have one of the following amino acid sequences:
SEQ ID NO: 19 - EISLSYSAGALASCMGLIYNRMGAVTTEVAFGLVCATCEQIADSQHRSHR
SEQ ID NO: 20 - EISLSYSAGALASCMGLIYNRMGAVTTEVAFGLVCATCEQIADSQHR

SEQ ID NO: 19 includes the complete Cys-Cys-His-His motif (residues 148-162 of SEQ ID NO: 2) that may give affinity for zinc [72]. Thus a matrix protein used with the invention may additionally include a zinc ion.

The SEQ ID NO: 18 sequence includes the sequence LSYSXGALA (SEQ ID NO: 1), which is very well conserved between influenza A virus strains, with the 5th residue 'X' being either Thr (LSYSTGALA, SEQ ID NO: 21) or Ala (LSYSAGALA, SEQ ID NO: 22). Variations of this conserved 9mer are known (*e.g.* in A/Swine/Ontario/01911-2/99 (H4N6) the 9mer is LNYSTGALA [GI:10442678; SEQ ID NO: 23], and in A/swine/England/191973/92 (H1N7) it is LGYSTGALA [GI:1835734; SEQ ID NO: 24], in A/Chicken/Pennsylvania/13609/93 (H5N2) it is LSYSTGALT [GI:4584948; SEQ ID NO: 25], in A/WSN/33 it is FSYSAGALA [GI:324407; SEQ ID NO: 26]), but SEQ ID NO: 1 is the most common sequence. The core YSXGAL (SEQ ID NO: 27) features in all of these sequences. Sequences in influenza viruses can conveniently be located in the Influenza Sequence Database (ISD) at *www.flu.lanl.gov* [73]. Further sequences can be found in reference 74.

Thus preferred matrix proteins included in compositions of the disclosure include one or more of the amino acid sequences SEQ ID NOs: 1, 21, 22, 23, 24, 25, 26 and/or 27. Whereas full-length M1 protein is a 27.8kDa protein, however, the matrix protein included in the compositions of the invention is has a molecular weight of <20kDa *e.g.* ≤15kDa, ≤12kDa, ≤10kDa, ≤9kDa, ≤8kDa, ≤7kDa, ≤6kDa, ≤5.5kDa, or about 5kDa. The molecular weight of the matrix protein preferably falls within the range of 2-8kDa *e.g.* 3-7kDa, 4-6kDa, or about 5kDa. The N-terminus of the matrix protein may be Glu-Ile-Ser followed by one of the amino acid sequences SEQ ID NOs: 1, 19, 20, 21, 22, 23, or 24.

The matrix protein may form oligomers (*e.g.* dimers, such as homodimers) within the composition.

If the matrix protein includes amino acid 137 (numbered as in SEQ ID NO: 2) then this amino acid may be Ala (as in SEQ ID NO: 2) or Thr (as seen in pathogenic H5N1 human viruses).

The matrix protein may be present in various amounts, but will typically be present at from 1µg/ml to 15µg/ml e.g. between 2-14µg/ml, between 3-13µg/ml, between 4-12µg/ml, between 5-11µg/ml, between 6-10µg/ml, between 7-9µg/ml, *etc.* Concentrations of less than 1µg/ml are also possible.

By including these matrix proteins in addition to haemagglutinin then compositions of the invention benefit from any T cell epitopes that are present, which may improve the cross-protection (within the same HA type and also between different HA types) elicited by a vaccine [75]. The matrix proteins are present endogenously as a result of composition preparation (*e.g.* it may co-purify with HA).

Without wishing to be bound by theory, the inventors believe that matrix protein may bind to HA in a vaccine to form a stable complex. If the complex is more stable than HA alone then the shelf-life of a vaccine may be improved, and in particular its ability to withstand storage outside a refrigerator.

The region around SEQ ID NO: 1 in M1 can act as a lipid-binding domain [76]. By including this region in a matrix protein, therefore, the protein can advantageously interact with fatty adjuvants, as described in more detail below.

Where a composition includes HA from more than one influenza A virus strain then it will generally also include matrix protein from more than one strain. Whereas the HA from the strains will usually be different from each other, however, the matrix proteins may be the same. If they are identical then, in the final product, it may not be possible to distinguish the two matrix proteins, but they will have different origins.

As well as including HA and matrix, compositions of the disclosure may include neuraminidase and/or nucleoprotein.

Where a composition includes HA from an influenza B virus then it may also include M1 protein from an influenza B virus.

### Host cell DNA

Where virus has been grown on a cell line then it is standard practice to minimize the amount of residual cell line DNA in the final vaccine, in order to minimize any oncogenic activity of the DNA. This safety measure is particularly important when including an influenza virus matrix protein in a vaccine, as the matrix protein can bind to nucleic acids (including RNA and double-stranded DNA) [77] and may thus retain DNA more readily than existing HA-based vaccines.

Thus the composition preferably contains less than lOng (preferably less than 1ng, and more preferably less than 100pg) of residual host cell DNA per dose, although trace amounts of host cell DNA may be present. It is preferred that the average length of any residual host cell DNA is less than 500bp *e.g.* less than 400bp, less than 300bp, less than 200bp, less than 100bp, *etc.* In general, the host cell DNA that it is desirable to exclude from compositions of the invention is DNA that is longer than 100bp.

Measurement of residual host cell DNA is now a routine regulatory requirement for biologicals and is within the normal capabilities of the skilled person. The assay used to measure DNA will typically be a validated assay [78,79]. The performance characteristics of a validated assay can be described in mathematical and quantifiable terms, and its possible sources of error will have been identified. The assay will generally have been tested for characteristics such as accuracy, precision, specificity. Once an assay has been calibrated (*e.g.* against known standard quantities of host cell DNA) and tested then quantitative DNA measurements can be routinely performed. Three principle techniques for DNA quantification can be used: hybridization methods, such as Southern blots or slot blots [80]; immunoassay methods, such as the Threshold™ System [81]; and quantitative PCR [82]. These methods are all familiar to the skilled person, although the precise characteristics of each method may depend on the host cell in question *e.g.* the choice of probes for hybridization, the choice of primers and/or probes for amplification, *etc.* The Threshold™ system from *Molecular Devices* is a quantitative assay for picogram levels of total DNA, and has been used for monitoring levels of contaminating DNA in biopharmaceuticals [81]. A typical assay involves non-sequence-specific formation of a reaction complex between a biotinylated ssDNA binding protein, a urease-conjugated anti-ssDNA antibody, and DNA. All assay components are included in the complete Total DNA Assay Kit available from the manufacturer. Various commercial manufacturers offer quantitative PCR assays for detecting residual host cell DNA *e.g.* AppTec™ Laboratory Services, BioReliance™, Althea Technologies, *etc.* A comparison of a chemiluminescent hybridisation assay and the total DNA Threshold™ system for measuring host cell DNA contamination of a human viral vaccine can be found in reference 83.

Contaminating DNA can be removed during vaccine preparation using standard purification procedures *e.g.* chromatography, *etc.* Removal of residual host cell DNA can be enhanced by nuclease treatment *e.g.* by using a DNase. A convenient method for reducing host cell DNA contamination is disclosed in references 84 & 85, involving a two-step treatment, first using a DNase (*e.g.* Benzonase), which may be used during viral growth, and then a cationic detergent (*e.g.* CTAB), which may be used during virion disruption. Treatment with an alkylating agent, such as β-propiolactone, can also be used to remove host cell DNA, and advantageously may also be used to inactivate virions [86].

Vaccines containing <10ng (*e.g.* <1ng, <100pg) host cell DNA per 15µg of haemagglutinin are preferred, as are vaccines containing <10ng (*e.g.* <1ng, <100pg) host cell DNA per 0.25ml volume. Vaccines containing <10ng (*e.g.* <1ng, <100pg) host cell DNA per 50µg of haemagglutinin are more preferred, as are vaccines containing <10ng (*e.g.* <1ng, <100pg) host cell DNA per 0.5ml volume.

### Adjuvants

Compositions of the disclosure may advantageously include an adjuvant, which can function to enhance the immune responses (humoral and/or cellular) elicited in a patient who receives the composition. The use of adjuvants with influenza vaccines has been described before. In references 87 & 88, aluminum hydroxide was used, and in reference 89, a mixture of aluminum hydroxide and aluminum phosphate was used. Reference 90 also described the use of aluminum salt adjuvants. The FLUAD™ product from Chiron Vaccines includes an oil-in-water emulsion.

Adjuvants that can be used with the invention include, but are not limited to:
- A mineral-containing composition, including calcium salts and aluminum salts (or mixtures thereof). Calcium salts include calcium phosphate (*e.g.* the "CAP" particles disclosed in ref. 91). Aluminum salts include hydroxides, phosphates, sulfates, *etc.,* with the salts taking any suitable form (*e.g.* gel, crystalline, amorphous, *etc.*). Adsorption to these salts is preferred. The mineral containing compositions may also be formulated as a particle of metal salt [92]. Aluminum salt adjuvants are described in more detail below.
- Cytokine-inducing agents (see in more detail below).
- Saponins [chapter 22 of ref. 128], which are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™. Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in ref. 93. Saponin formulations may also comprise a sterol, such as cholesterol [94]. Combinations of saponins and cholesterols can be used to form unique particles called immunostimulating complexs (ISCOMs) [chapter 23 of ref. 128]. ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in refs. 94-96. Optionally, the ISCOMS may be devoid of additional detergent [97]. A review of the development of saponin based adjuvants can be found in refs. 98 & 99.
- Fatty adjuvants (see in more detail below), including oil-in-water emulsions.
- Bacterial ADP-ribosylating toxins (*e.g.* the *E.coli* heat labile enterotoxin "LT", cholera toxin "CT", or pertussis toxin "PT") and detoxified derivatives thereof, such as the mutant toxins known as LT-K63 and LT-R72 [100]. The use of detoxified ADP-ribosylating toxins as mucosal adjuvants is described in ref. 101 and as parenteral adjuvants in ref. 102.
- Bioadhesives and mucoadhesives, such as esterified hyaluronic acid microspheres [103] or chitosan and its derivatives [104].
- Microparticles (*i.e*. a particle of ∼100nm to ∼150µm in diameter, more preferably ∼200nm to ∼30µm in diameter, or ∼500nm to ∼10µm in diameter) formed from materials that are biodegradable and non-toxic (*e.g*. a poly(α-hydroxy acid), a polyhydroxybutyric acid, a polyorthoester, a polyanhydride, a polycaprolactone, *etc.*), with poly(lactide-co-glycolide) being preferred, optionally treated to have a negatively-charged surface (*e.g.* with SDS) or a positively-charged surface (*e.g.* with a cationic detergent, such as CTAB).
- Liposomes (Chapters 13 & 14 of ref. 128). Examples of liposome formulations suitable for use as adjuvants are described in refs. 105-107.
- Polyoxyethylene ethers and polyoxyethylene esters [108]. Such formulations further include polyoxyethylene sorbitan ester surfactants in combination with an octoxynol [109] as well as polyoxyethylene alkyl ethers or ester surfactants in combination with at least one additional non-ionic surfactant such as an octoxynol [110]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.
- Muramyl peptides, such as N-acetylmuramyl-L-threonyl-D-isoglutamine ("thr-MDP"), N-acetyl-normuramyl-L-alanyl-D-isoglutamine (nor-MDP), N-acetylglucsaminyl-N-acetylmuramyl-L-Al-D-isoglu-L-Ala-dipalmitoxy propylamide ("DTP-DPP", or "Theramide™), N-acetylmuramyl-L-alanyl-D-isoglutaminyl-L-alanine-2-(1'-2'dipalmitoyl-sn-glycero-3-hydroxyphosphoryloxy)-ethylamine ("MTP-PE").
- An outer membrane protein proteosome preparation prepared from a first Gram-negative bacterium in combination with a liposaccharide preparation derived from a second Gram-negative bacterium, wherein the outer membrane protein proteosome and liposaccharide preparations form a stable non-covalent adjuvant complex. Such complexes include "IVX-908", a complex comprised of *Neisseria meningitidis* outer membrane and lipopolysaccharides. They have been used as adjuvants for influenza vaccines [111].
- A polyoxidonium polymer [112,113] or other N-oxidized polyethylene-piperazine derivative.
- Methyl inosine 5'-monophosphate ("MIMP") [114].
- A polyhydroxlated pyrrolizidine compound [115], such as one having formula: 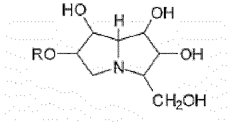 where R is selected from the group comprising hydrogen, straight or branched, unsubstituted or substituted, saturated or unsaturated acyl, alkyl (*e.g.* cycloalkyl), alkenyl, alkynyl and aryl groups, or a pharmaceutically acceptable salt or derivative thereof. Examples include, but are not limited to: casuarine, casuarine-6-α-D-glucopyranose, 3-*epi*-casuarine, 7-*epi*-casuarine, 3,7-di*epi*-casuarine, *etc.*
- A CD1d ligand, such as an α-glycosylceramide [116-123] (*e.g*. α-galactosylceramide), phytosphingosine-containing α-glycosylceramides, OCH, KRN7000 [(2S,3S,4R)-1-0-(α-D-galactopyranosyl)-2-(N-hexacosanoylamino)-1,3,4-octadecanetriol], CRONY-101, 3"-O-sulfo-galactosylceramide, *etc.*
- A gamma inulin [124] or derivative thereof, such as algammulin.

These and other adjuvant-active substances are discussed in more detail in references 128 & 129.

Compositions may include two or more of said adjuvants. For example, they may advantageously include both an oil-in-water emulsion and a cytokine-inducing agent, as this combination improves the cytokine responses elicited by influenza vaccines, such as the interferon-γ response, with the improvement being much greater than seen when either the emulsion or the agent is used on its own.

Antigens and adjuvants in a composition will typically be in admixture.

### Oil-in-water emulsion adjuvants

Oil-in-water emulsions have been found to be particularly suitable for use in adjuvanting influenza virus vaccines. Various such emulsions are known, and they typically include at least one oil and at least one surfactant, with the oil(s) and surfactant(s) being biodegradable (metabolisable) and biocompatible. The oil droplets in the emulsion are generally less than 5µm in diameter, and may even have a sub-micron diameter, with these small sizes being achieved with a microfluidiser to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used e.g. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol™ NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Non-ionic surfactants are preferred. Preferred surfactants for including in the emulsion are Tween 80 (polyoxyethylene sorbitan monooleate), Span 85 (sorbitan trioleate), lecithin and Triton X-100.

Mixtures of surfactants can be used *e.g.* Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Specific oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [125-127], as described in more detail in Chapter 10 of ref. 128 and chapter 12 of ref. 129. The MF59 emulsion advantageously includes citrate ions *e.g.* 10mM sodium citrate buffer.
- An emulsion of squalene, a tocopherol, and Tween 80. The emulsion may include phosphate buffered saline. It may also include Span 85 (*e.g.* at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. Squalene and Tween 80 may be present volume ratio of about 5:2. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets *e.g.* with an average diameter of between 100 and 250nm, preferably about 180nm.
- An emulsion of squalene, a tocopherol, and a Triton detergent (*e.g.* Triton X-100). The emulsion may also include a 3d-MPL (see below). The emulsion may contain a phosphate buffer.
- An emulsion comprising a polysorbate *(e.g.* polysorbate 80), a Triton detergent *(e.g.* Triton X-100) and a tocopherol (*e.g.* an α-tocopherol succinate). The emulsion may include these three components at a mass ratio of about 75:11:10 (*e.g.* 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [130] (0.05-1% Thr-MDP, 5% squalane, 2.5% Pluronic L121 and 0.2% polysorbate 80). It can also be used without the Thr-MDP, as in the "AF" adjuvant [131] (5% squalane, 1.25% Pluronic L121 and 0.2% polysorbate 80). Microfluidisation is preferred.
- An emulsion having from 0.5-50% of an oil, 0.1-10% of a phospholipid, and 0.05-5% of a non-ionic surfactant. As described in reference 132, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 133, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion in which a saponin (*e.g.* QuilA or QS21) and a sterol (*e.g.* a cholesterol) are associated as helical micelles [134].

The emulsions may be mixed with antigen extemporaneously, at the time of delivery. Thus the adjuvant and antigen may be kept separately in a packaged or distributed vaccine, ready for final formulation at the time of use. The antigen will generally be in an aqueous form, such that the vaccine is finally prepared by mixing two liquids. The volume ratio of the two liquids for mixing can vary (*e.g.* between 5:1 and 1:5) but is generally about 1:1.

After the antigen and adjuvant have been mixed, haemagglutinin antigen will generally remain in aqueous solution but may distribute itself around the oil/water interface. In general, little if any haemagglutinin will enter the oil phase of the emulsion.

Where a composition includes a tocopherol, any of the α, β, γ, δ, ε or ξ tocopherols can be used, but α-tocopherols are preferred. The tocopherol can take several forms e.g. different salts and/or isomers. Salts include organic salts, such as succinate, acetate, nicotinate, *etc.* D-α-tocopherol and DL-α-tocopherol can both be used. Tocopherols are advantageously included in vaccines for use in elderly patients (*e.g.* aged 60 years or older) because vitamin E has been reported to have a positive effect on the immune response in this patient group [135]. They also have antioxidant properties that may help to stabilize the emulsions [136]. A preferred α-tocopherol is DL-α-tocopherol, and the preferred salt of this tocopherol is the succinate. The succinate salt has been found to cooperate with TNF-related ligands *in vivo.* Moreover, α-tocopherol succinate is known to be compatible with influenza vaccines and to be a useful preservative as an alternative to mercurial compounds [9].

### Cytokine-inducing agents

Cytokine-inducing agents for inclusion in compositions of the disclosure are able, when administered to a patient, to elicit the immune system to release cytokines, including interferons and interleukins. Cytokine responses are known to be involved in the early and decisive stages of host defense against influenza infection [137]. Preferred agents can elicit the release of one or more of: interferon-y; interleukin-1; interleukin-2; interleukin-12; TNF-α; TNF-β; and GM-CSF. Preferred agents elicit the release of cytokines associated with a Th1-type immune response *e.g.* interferon-γ, TNF-α, interleukin-2. Stimulation of both interferon-γ and interleukin-2 is preferred.

As a result of receiving a composition of the disclosure, therefore, a patient will have T cells that, when stimulated with an influenza antigen, will release the desired cytokine(s) in an antigen-specific manner. For example, T cells purified form their blood will release γ-interferon when exposed *in vitro* to influenza virus haemagglutinin. Methods for measuring such responses in peripheral blood mononuclear cells (PBMC) are known in the art, and include ELISA, ELISPOT, flow-cytometry and real-time PCR. For example, reference 138 reports a study in which antigen-specific T cell-mediated immune responses against tetanus toxoid, specifically γ-interferon responses, were monitored, and found that ELISPOT was the most sensitive method to discriminate antigen-specific TT-induced responses from spontaneous responses, but that intracytoplasmic cytokine detection by flow cytometry was the most efficient method to detect re-stimulating effects.

Suitable cytokine-inducing agents include, but are not limited to:
- An immunostimulatory oligonucleotide, such as one containing a CpG motif (a dinucleotide sequence containing an unmethylated cytosine linked by a phosphate bond to a guanosine), or a double-stranded RNA, or an oligonucleotide containing a palindromic sequence, or an oligonucleotide containing a poly(dG) sequence.
- 3-O-deacylated monophosphoryl lipid A ('3dMPL', also known as 'MPL™') [139-142].
- An imidazoquinoline compound, such as Imiquimod ("R-837") [143,144], Resiquimod ("R-848") [145], and their analogs; and salts thereof (*e.g.* the hydrochloride salts). Further details about immunostimulatory imidazoquinolines can be found in references 146 to 150.
- A thiosemicarbazone compound, such as those disclosed in reference 151. Methods of formulating, manufacturing, and screening for active compounds are also described in reference 151. The thiosemicarbazones are particularly effective in the stimulation of human peripheral blood mononuclear cells for the production of cytokines, such as TNF-α.
- A tryptanthrin compound, such as those disclosed in reference 152. Methods of formulating, manufacturing, and screening for active compounds are also described in reference 152. The thiosemicarbazones are particularly effective in the stimulation of human peripheral blood mononuclear cells for the production of cytokines, such as TNF-α.
- A nucleoside analog, such as: (a) Isatorabine (ANA-245; 7-thia-8-oxoguanosine): 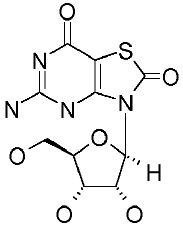 and prodrugs thereof; (b) ANA975; (c) ANA-025-1; (d) ANA380; (e) the compounds disclosed in references 153 to 155; (f) a compound having the formula: 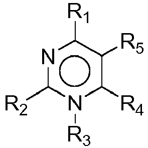 wherein:
   R₁ and R₂ are each independently H, halo, -NRₐR_{b}, -OH, C₁₋₆ alkoxy, substituted C₁₋₆ alkoxy, heterocyclyl, substituted heterocyclyl, C₆₋₁₀ aryl, substituted C₆₋₁₀ aryl, C₁₋₆ alkyl, or substituted C₁₋₆ alkyl;
   R₃ is absent, H, C₁₋₆ alkyl, substituted C₁₋₆ alkyl, C₆₋₁₀ aryl, substituted C₆₋₁₀ aryl, heterocyclyl, or substituted heterocyclyl;
   R₄ and R₅ are each independently H, halo, heterocyclyl, substituted heterocyclyl, -C(O)-R_{d}, C₁₋₆ alkyl, substituted C₁₋₆ alkyl, or bound together to form a 5 membered ring as in R₄₋₅: 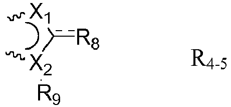 the binding being achieved at the bonds indicated by a 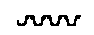
   X₁ and X₂ are each independently N, C, O, or S;
   R₈ is H, halo, -OH, C₁₋₆ alkyl, C₂₋₆ alkenyl, C₂₋₆ alkynyl, -OH, -NRₐR_{b}, -(CH₂)ₙ-O-R_{c}, -O-(C₁₋₆ alkyl), -S(O)ₚRₑ, or -C(O)-R_{d};
   R₉ is H, C₁₋₆ alkyl, substituted C₁₋₆ alkyl, heterocyclyl, substituted heterocyclyl or R₉ₐ, wherein R₉ₐ is: 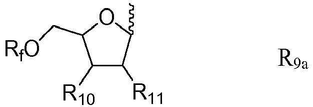 the binding being achieved at the bond indicated by a 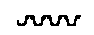
   R₁₀ and R₁₁ are each independently H, halo, C₁₋₆ alkoxy, substituted C₁₋₆ alkoxy,-NRₐR_{b}, or -OH;
   each Rₐ and R_{b} is independently H, C₁₋₆ alkyl, substituted C₁₋₆ alkyl, -C(O)R_{d}, C₆₋₁₀ aryl;
   each R_{c} is independently H, phosphate, diphosphate, triphosphate, C₁₋₆ alkyl, or substituted C₁₋₆ alkyl;
   each R_{d} is independently H, halo, C₁₋₆ alkyl, substituted C₁₋₆ alkyl, C₁₋₆ alkoxy, substituted C₁₋₆ alkoxy, -NH₂, -NH(C₁₋₆ alkyl), -NH(substituted C₁₋₆ alkyl), -N(C₁₋₆ alkyl)₂, -N(substituted C₁₋₆ alkyl)₂, C₆₋₁₀ aryl, or heterocyclyl;
   each Rₑ is independently H, C₁₋₆ alkyl, substituted C₁₋₆ alkyl, C₆₋₁₀ aryl, substituted C₆₋₁₀ aryl, heterocyclyl, or substituted heterocyclyl;
   each R_{f} is independently H, C₁₋₆ alkyl, substituted C₁₋₆ alkyl, -C(O)R_{d}, phosphate, diphosphate, or triphosphate;
   each n is independently 0, 1, 2, or 3;
   each p is independently 0, 1, or 2; or
   or (g) a pharmaceutically acceptable salt of any of (a) to (f), a tautomer of any of (a) to (f), or a pharmaceutically acceptable salt of the tautomer.
- Loxoribine (7-allyl-8-oxoguanosine) [156].
- Compounds disclosed in reference 157, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [158,159], Hydrapthalamide compounds, Benzophenone compounds, Isoxazole compounds, Sterol compounds, Quinazilinone compounds, Pyrrole compounds [160], Anthraquinone compounds, Quinoxaline compounds, Triazine compounds, Pyrazalopyrimidine compounds, and Benzazole compounds [161].
- Compounds disclosed in reference 162.
- An aminoalkyl glucosaminide phosphate derivative, such as RC-529 [163,164].
- A phosphazene, such as poly[di(carboxylatophenoxy)phosphazene] ("PCPP") as described, for example, in references 165 and 166.
- Small molecule immunopotentiators (SMIPs) such as:
   N2-methyl-1-(2-methylpropyl)-1H-imidazo[4,5-c]quinoline-2,4-diamine
   N2,N2-dimethyl-1-(2-methylpropyl)-1H-imidazo[4,5-c]quinoline-2,4-diamine
   N2-ethyl-N2-methyl-1-(2-methylpropyl)-1H-imidazo[4,5-c]quinoline-2,4-diamine
   N2-methyl-1-(2-methylpropyl)-N2-propyl-1H-imidazo[4,5-c]quinoline-2,4-diamine
   1-(2-methylpropyl)-N2-propyl-1H-imidazo[4,5-c]quinoline-2,4-diamine
   N2-butyl-1-(2-methylpropyl)-1H-imidazo[4,5-c]quinoline-2,4-diamine
   N2-butyl-N2-methyl-1-(2-methylpropyl)-1H-imidazo[4,5-c]quinoline-2,4-diamine
   N2-methyl-1-(2-methylpropyl)-N2-pentyl-1H-imidazo[4,5-c]quinoline-2,4-diamine
   N2-methyl-1-(2-methylpropyl)-N2-prop-2-enyl-1H-imidazo[4,5-c]quinoline-2,4-diamine
   1-(2-methylpropyl)-2-[(phenylmethyl)thio]-1H-imidazo[4,5-c]quinolin-4-amine
   1-(2-methylpropyl)-2-(propylthio)-1H-imidazo[4,5-c]quinolin-4-amine
   2-[[4-amino-1-(2-methylpropyl)-1H-imidazo[4,5-c]quinolin-2-yl](methyl)amino]ethanol
   2-[[4-amino-1-(2-methylpropyl)-1H-imidazo[4,5-c]quinolin-2-yl](methyl)amino]ethyl acetate
   4-amino-1-(2-methylpropyl)-1,3-dihydro-2H-imidazo[4,5-c]quinolin-2-one
   N2-butyl-1-(2-methylpropyl)-N4,N4-bis(phenylmethyl)-1H-imidazo[4,5-c]quinoline-2,4-diamine
   N2-butyl-N2-methyl-1-(2-methylpropyl)-N4,N4-bis(phenylmethyl)-1H-imidazo[4,5-c]quinoline-2,4-diamine
   N2-methyl-1-(2-methylpropyl)-N4,N4-bis(phenylmethyl)-1H-imidazo[4,5-c]quinoline-2,4-diamine
   N2,N2-dimethyl-1-(2-methylpropyl)-N4,N4-bis(phenylmethyl)-1H-imidazo[4,5-c]quinoline-2,4-diamine
   1-{4-amino-2-[methyl(propyl)amino]-1H-imidazo[4,5-c]quinolin-1-yl}-2-methylpropan-2-ol
   1-[4-amino-2-(propylamino)-1H-imidazo[4,5-c]quinolin-1-yl]-2-methylpropan-2-ol
   N4,N4-dibenzyl-1-(2-methoxy-2-methylpropyl)-N2-propyl-1H-imidazo[4,5-c]quinoline-2,4-diamine.

The cytokine-inducing agents for use in the present invention may be modulators and/or agonists of Toll-Like Receptors (TLR). For example, they may be agonists of one or more of the human TLR1, TLR2, TLR3, TLR4, TLR7, TLR8, and/or TLR9 proteins. Preferred agents are agonists of TLR7 (*e.g.* imidazoquinolines) and/or TLR9 (*e.g.* CpG oligonucleotides). These agents are useful for activating innate immunity pathways.

The cytokine-inducing agent can be added to the composition at various stages during its production. For example, it may be within an antigen composition, and this mixture can then be added to an oil-in-water emulsion. As an alternative, it may be within an oil-in-water emulsion, in which case the agent can either be added to the emulsion components before emulsification, or it can be added to the emulsion after emulsification. Similarly, the agent may be coacervated within the emulsion droplets. The location and distribution of the cytokine-inducing agent within the final composition will depend on its hydrophilic/lipophilic properties *e.g.* the agent can be located in the aqueous phase, in the oil phase, and/or at the oil-water interface.

The cytokine-inducing agent can be conjugated to a separate agent, such as an antigen (*e.g.* CRM197). A general review of conjugation techniques for small molecules is provided in ref. 167. As an alternative, the adjuvants may be non-covalently associated with additional agents, such as by way of hydrophobic or ionic interactions.

Two preferred cytokine-inducing agents are (a) immunostimulatory oligonucleotides and (b) 3dMPL.

Immunostimulatory oligonucleotides can include nucleotide modifications/analogs such as phosphorothioate modifications and can be double-stranded or (except for RNA) single-stranded. References 168, 169 and 170 disclose possible analog substitutions *e.g.* replacement of guanosine with 2'-deoxy-7-deazaguanosine. The adjuvant effect of CpG oligonucleotides is further discussed in refs. 171-176. A CpG sequence may be directed to TLR9, such as the motif GTCGTT or TTCGTT [177]. The CpG sequence may be specific for inducing a Th1 immune response, such as a CpG-A ODN (oligodeoxynucleotide), or it may be more specific for inducing a B cell response, such a CpG-B ODN. CpG-A and CpG-B ODNs are discussed in refs. 178-180. Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, references 177 & 181-183. A useful CpG adjuvant is CpG7909, also known as ProMune™ (Coley Pharmaceutical Group, Inc.).

As an alternative, or in addition, to using CpG sequences, TpG sequences can be used [184]. These oligonucleotides may be free from unmethylated CpG motifs.

The immunostimulatory oligonucleotide may be pyrimidine-rich. For example, it may comprise more than one consecutive thymidine nucleotide (*e.g.* TTTT, as disclosed in ref. 184), and/or it may have a nucleotide composition with >25% thymidine (*e.g.* >35%, >40%, >50%, >60%, >80%, *etc.*)*.* For example, it may comprise more than one consecutive cytosine nucleotide (*e.g.* CCCC, as disclosed in ref. 184), and/or it may have a nucleotide composition with >25% cytosine (*e.g.* >35%, >40%, >50%, >60%, >80%, *etc.*). These oligonucleotides may be free from unmethylated CpG motifs.

Immunostimulatory oligonucleotides will typically comprise at least 20 nucleotides. They may comprise fewer than 100 nucleotides.

3dMPL (also known as 3 de-O-acylated monophosphoryl lipid A or 3-O-desacyl-4'-monophosphoryl lipid A) is an adjuvant in which position 3 of the reducing end glucosamine in monophosphoryl lipid A has been de-acylated. 3dMPL has been prepared from a heptoseless mutant of *Salmonella minnesota*, and is chemically similar to lipid A but lacks an acid-labile phosphoryl group and a base-labile acyl group. It activates cells of the monocyte/macrophage lineage and stimulates release of several cytokines, including IL-1, IL-12, TNF-α and GM-CSF (see also ref. 185). Preparation of 3dMPL was originally described in reference 186.

3dMPL can take the form of a mixture of related molecules, varying by their acylation (*e.g.* having 3, 4, 5 or 6 acyl chains, which may be of different lengths). The two glucosamine (also known as 2-deoxy-2-amino-glucose) monosaccharides are N-acylated at their 2-position carbons *(i.e.* at positions 2 and 2'), and there is also O-acylation at the 3' position. The group attached to carbon 2 has formula -NH-CO-CH₂-CR¹R^{1'}. The group attached to carbon 2' has formula -NH-CO-CH₂-CR²R^{2'}. The group attached to carbon 3' has formula -O-CO-CH₂-CR³R^{3'}. A representative structure is:

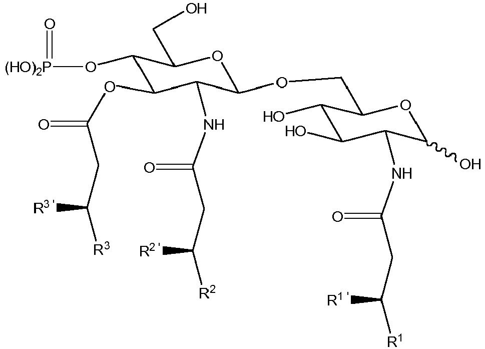

Groups R¹, R² and R³ are each independently -(CH₂)ₙ-CH₃. The value of *n* is preferably between 8 and 16, more preferably between 9 and 12, and is most preferably 10.

Groups R^{1'}, R^{2'} and R^{3'} can each independently be: (a) -H; (b) -OH; or (c) -O-CO-R⁴, where R⁴ is either -H or -(CH₂)ₘ-CH₃, wherein the value of *m* is preferably between 8 and 16, and is more preferably 10, 12 or 14. At the 2 position, *m* is preferably 14. At the 2' position, *m* is preferably 10. At the 3' position, *m* is preferably 12. Groups R^{1'}, R^{2'} and R^{3'} are thus preferably -O-acyl groups from dodecanoic acid, tetradecanoic acid or hexadecanoic acid.

When all of R^{1'}, R^{2'} and R³ are -H then the 3dMPL has only 3 acyl chains (one on each of positions 2, 2' and 3'). When only two of R^{1'}, R^{2'} and R^{3'} are -H then the 3dMPL can have 4 acyl chains. When only one of R^{1'}, R^{2'} and R^{3'} is -H then the 3dMPL can have 5 acyl chains. When none of R^{1'}, R^{2'} and R^{3'} is -H then the 3dMPL can have 6 acyl chains. The 3dMPL adjuvant used according to the invention can be a mixture of these forms, with from 3 to 6 acyl chains, but it is preferred to include 3dMPL with 6 acyl chains in the mixture, and in particular to ensure that the hexaacyl chain form makes up at least 10% by weight of the total 3dMPL *e.g.* ≥20%, ≥30%, ≥40%, ≥50% or more. 3dMPL with 6 acyl chains has been found to be the most adjuvant-active form.

Thus the most preferred form of 3dMPL for inclusion in compositions of the disclosure has formula (IV), shown below.

Where 3dMPL is used in the form of a mixture then references to amounts or concentrations of 3dMPL in compositions of the disclosure refer to the combined 3dMPL species in the mixture.

In aqueous conditions, 3dMPL can form micellar aggregates or particles with different sizes *e.g.* with a diameter <150nm or >500nm. Either or both of these can be used with the invention, and the better particles can be selected by routine assay. Smaller particles (*e.g.* small enough to give a clear aqueous suspension of 3dMPL) are preferred for use according to the invention because of their superior activity [187]. Preferred particles have a mean diameter less than 220nm, more preferably less than 200nm or less than 150nm or less than 120nm, and can even have a mean diameter less than 100nm. In most cases, however, the mean diameter will not be lower than 50nm. These particles are small enough to be suitable for filter sterilization. Particle diameter can be assessed by the routine technique of dynamic light scattering, which reveals a mean particle diameter. Where a particle is said to have a diameter of *x* nm, there will generally be a distribution of particles about this mean, but at least 50% by number (*e.g.* ≥60%, ≥70%, ≥80%, ≥90%, or more) of the particles will have a diameter within the range *x*±25%.

3dMPL can advantageously be used in combination with an oil-in-water emulsion. Substantially all of the 3dMPL may be located in the aqueous phase of the emulsion.

The 3dMPL can be used on its own, or in combination with one or more further compounds. For example, it is known to use 3dMPL in combination with the QS21 saponin [188] (including in an oil-in-water emulsion [189]), with an immunostimulatory oligonucleotide, with both QS21 and an immunostimulatory oligonucleotide, with aluminum phosphate [190], with aluminum hydroxide [191], or with both aluminum phosphate and aluminum hydroxide.

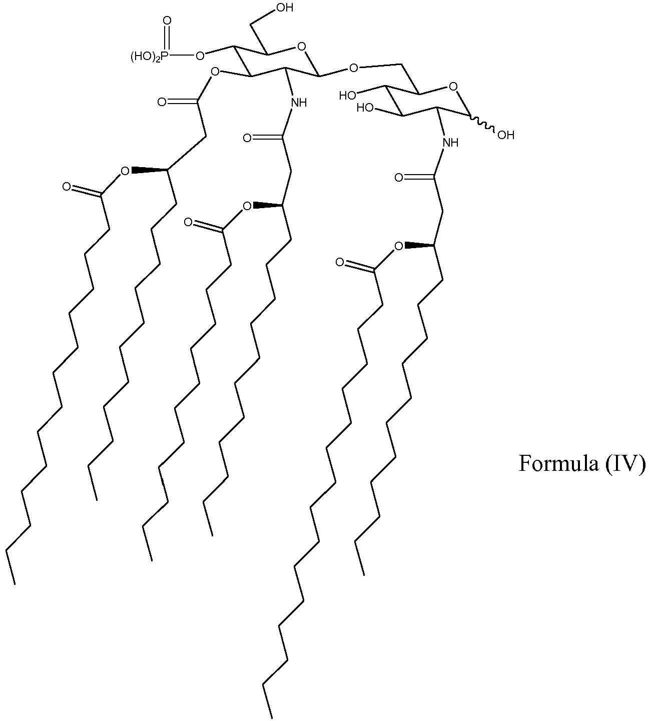

### Fatty adjuvants

Fatty adjuvants that can be used with the invention include the oil-in-water emulsions described above, and also include, for example:
- A compound of formula I, II or III, or a salt thereof: 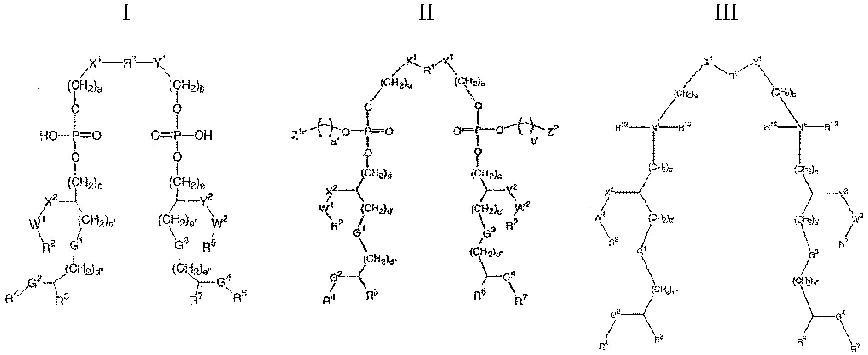 as defined in reference 192, such as 'ER 803058', 'ER 803732', 'ER 804053', ER 804058', 'ER 804059', 'ER 804442', 'ER 804680', 'ER 804764', ER 803022 or 'ER 804057' *e.g*.: 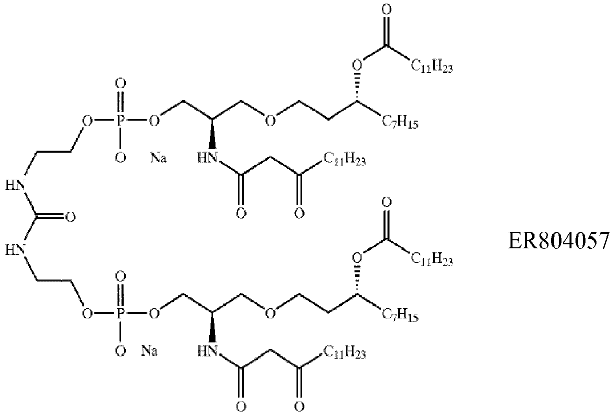 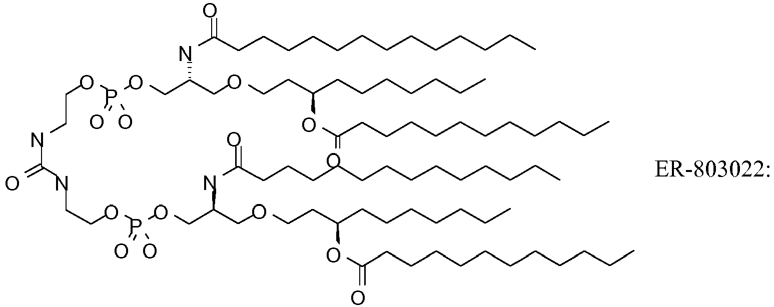
- Derivatives of lipid A from *Escherichia coli* such as OM-174 (described in refs. 193 & 194).
- A formulation of a cationic lipid and a (usually neutral) co-lipid, such as aminopropyl-dimethyl-myristoleyloxy-propanaminium bromide-diphytanoylphosphatidyl-ethanolamine ("Vaxfectin™") or aminopropyl-dimethyl-bis-dodecyloxy-propanaminium bromide-dioleoylphosphatidyl-ethanolamine ("GAP-DLRIE:DOPE"). Formulations containing (±)-N-(3-aminopropyl)-N,N-dimethyl-2,3-bis(syn-9-tetradeceneyloxy)-1-propanaminium salts are preferred [195].
- 3-O-deacylated monophosphoryl lipid A (see above).
- Compounds containing lipids linked to a phosphate-containing acyclic backbone, such as the TLR4 antagonist E5564 [196,197]:

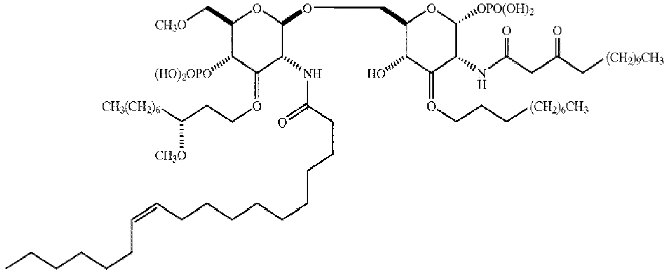

### Aluminum salt adjuvants

The adjuvants known as aluminum hydroxide and aluminum phosphate may be used. These names are conventional, but are used for convenience only, as neither is a precise description of the actual chemical compound which is present (*e.g.* see chapter 9 of reference 128). The invention can use any of the "hydroxide" or "phosphate" adjuvants that are in general use as adjuvants.

The adjuvants known as "aluminium hydroxide" are typically aluminium oxyhydroxide salts, which are usually at least partially crystalline. Aluminium oxyhydroxide, which can be represented by the formula AIO(OH), can be distinguished from other aluminium compounds, such as aluminium hydroxide Al(OH)₃, by infrared (IR) spectroscopy, in particular by the presence of an adsorption band at 1070cm⁻¹ and a strong shoulder at 3090-3100cm⁻¹ [chapter 9 of ref. 128]. The degree of crystallinity of an aluminium hydroxide adjuvant is reflected by the width of the diffraction band at half height (WHH), with poorly-crystalline particles showing greater line broadening due to smaller crystallite sizes. The surface area increases as WHH increases, and adjuvants with higher WHH values have been seen to have greater capacity for antigen adsorption. A fibrous morphology (*e.g.* as seen in transmission electron micrographs) is typical for aluminium hydroxide adjuvants. The pI of aluminium hydroxide adjuvants is typically about 11 i. *e.* the adjuvant itself has a positive surface charge at physiological pH. Adsorptive capacities of between 1.8-2.6 mg protein per mg Al⁺⁺⁺ at pH 7.4 have been reported for aluminium hydroxide adjuvants.

The adjuvants known as "aluminium phosphate" are typically aluminium hydroxyphosphates, often also containing a small amount of sulfate (*i.e.* aluminium hydroxyphosphate sulfate). They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 1.2. Hydroxyphosphates can be distinguished from strict AlPO₄ by the presence of hydroxyl groups. For example, an IR spectrum band at 3164cm⁻¹ (*e.g.* when heated to 200°C) indicates the presence of structural hydroxyls [ch.9 of ref. 128]

The PO₄/Al³⁺ molar ratio of an aluminium phosphate adjuvant will generally be between 0.3 and 1.2, preferably between 0.8 and 1.2, and more preferably 0.95+0.1. The aluminium phosphate will generally be amorphous, particularly for hydroxyphosphate salts. A typical adjuvant is amorphous aluminium hydroxyphosphate with PO₄/Al molar ratio between 0.84 and 0.92, included at 0.6mg Al³⁺/ml. The aluminium phosphate will generally be particulate (*e.g.* plate-like morphology as seen in transmission electron micrographs). Typical diameters of the particles are in the range 0.5-20µm (*e.g*. about 5-10µm after any antigen adsorption. Adsorptive capacities of between 0.7-1.5 mg protein per mg Al⁺⁺⁺ at pH 7.4 have been reported for aluminium phosphate adjuvants.

The point of zero charge (PZC) of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 *e.g.* about 5.7.

Suspensions of aluminium salts used to prepare compositions of the invention may contain a buffer (*e.g.* a phosphate or a histidine or a Tris buffer), but this is not always necessary. The suspensions are preferably sterile and pyrogen-free. A suspension may include free aqueous phosphate ions *e.g.* present at a concentration between 1.0 and 20 mM, preferably between 5 and 15 mM, and more preferably about 10 mM. The suspensions may also comprise sodium chloride.

The invention can use a mixture of both an aluminium hydroxide and an aluminium phosphate [89]. In this case there may be more aluminium phosphate than hydroxide *e.g.* a weight ratio of at least 2:1 *e.g.* ≥5:1, ≥6:1, ≥7:1, ≥8:1, ≥9:1, *etc.*

The concentration of Al⁺⁺⁺ in a composition for administration to a patient is preferably less than 10mg/ml *e.g.* ≤5 mg/ml, ≤4 mg/ml, ≤3 mg/ml, ≤2 mg/ml, ≤1 mg/ml, *etc.* A preferred range is between 0.3 and 1mg/ml. A maximum of 0.85mg/dose is preferred.

As well as including one or more aluminium salt adjuvants, the adjuvant component may include one or more further adjuvant or immunostimulating agents. Such additional components include, but are not limited to: a 3-O-deacylated monophosphoryl lipid A adjuvant ('3d-MPL'); and/or an oil-in-water emulsion. 3d-MPL has also been referred to as 3 de-O-acylated monophosphoryl lipid A or as 3-O-desacyl-4'-monophosphoryl lipid A. The name indicates that position 3 of the reducing end glucosamine in monophosphoryl lipid A is de-acylated. It has been prepared from a heptoseless mutant of *S.minnesota,* and is chemically similar to lipid A but lacks an acid-labile phosphoryl group and a base-labile acyl group. It activates cells of the monocyte/macrophage lineage and stimulates release of several cytokines, including IL-1, IL-12, TNF-α and GM-CSF. Preparation of 3d-MPL was originally described in reference 186, and the product has been manufactured and sold by Corixa Corporation under the name MPL™. Further details can be found in refs 139 to 142.

### Pharmaceutical compositions

Compositions of the invention are pharmaceutically acceptable. They usually include components in addition to the antigens e.g. they typically include one or more pharmaceutical carrier(s) and/or excipient(s). A thorough discussion of such components is available in reference 198.

Compositions will generally be in aqueous form.

The composition may include preservatives such as thiomersal or 2-phenoxyethanol. It is preferred, however, that the vaccine should be substantially free from (*i.e*. less than 5µg/ml) mercurial material *e.g.* thiomersal-free [9,199]. Vaccines containing no mercury are more preferred. Preservative-free vaccines are particularly preferred.

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, *etc.*

Compositions will generally have an osmolality of between 200 mOsm/kg and 400 mOsm/kg, preferably between 240-360 mOsm/kg, and will more preferably fall within the range of 290-310 mOsm/kg. Osmolality has previously been reported not to have an impact on pain caused by vaccination [200], but keeping osmolality in this range is nevertheless preferred.

Compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range.

The pH of a composition will generally be between 5.0 and 8.1, and more typically between 6.0 and 8.0 *e.g.* 6.5 and 7.5, or between 7.0 and 7.8. A process of the disclosure may therefore include a step of adjusting the pH of the bulk vaccine prior to packaging.

The composition is preferably sterile. The composition is preferably non-pyrogenic *e.g.* containing <1 EU (endotoxin unit, a standard measure) per dose, and preferably <0.1 EU per dose. The composition is preferably gluten free.

Compositions of the invention may include detergent *e.g.* a polyoxyethylene sorbitan ester surfactant (known as 'Tweens'), an octoxynol (such as octoxynol-9 (Triton X-100) or t-octylphenoxypolyethoxyethanol), a cetyl trimethyl ammonium bromide ('CTAB'), or sodium deoxycholate, particularly for a split or surface antigen vaccine. The detergent may be present only at trace amounts. Thus the vaccine may included less than 1mg/ml of each of octoxynol-10 and polysorbate 80. Other residual components in trace amounts could be antibiotics (*e.g.* neomycin, kanamycin, polymyxin B).

The composition may include material for a single immunisation, or may include material for multiple immunisations (*i.e*. a 'multidose' kit). The inclusion of a preservative is preferred in multidose arrangements. As an alternative (or in addition) to including a preservative in multidose compositions, the compositions may be contained in a container having an aseptic adaptor for removal of material.

Influenza vaccines are typically administered in a dosage volume of about 0.5ml, although a half dose (*i.e*. about 0.25ml) may be administered to children.

Compositions and kits are preferably stored at between 2°C and 8°C. They should not be frozen. They should ideally be kept out of direct light.

### Kits of the disclosure

Compositions of the invention may be prepared extemporaneously, at the time of delivery, particularly when an adjuvant is being used. Thus the disclosure includes kits including the various components ready for mixing. The kit allows the adjuvant and the antigen to be kept separately until the time of use. This arrangement is particularly useful when using an oil-in-water emulsion adjuvant.

The components are physically separate from each other within the kit, and this separation can be achieved in various ways. For instance, the two components may be in two separate containers, such as vials. The contents of the two vials can then be mixed *e.g.* by removing the contents of one vial and adding them to the other vial, or by separately removing the contents of both vials and mixing them in a third container.

In a preferred arrangement, one of the kit components is in a syringe and the other is in a container such as a vial. The syringe can be used (*e.g.* with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration.

In another preferred arrangement, the two kit components are held together but separately in the same syringe *e.g.* a dual-chamber syringe, such as those disclosed in references 201-208 *etc.* When the syringe is actuated (*e.g.* during administration to a patient) then the contents of the two chambers are mixed. This arrangement avoids the need for a separate mixing step at the time of use.

The kit components will generally be in aqueous form. In some arrangements, a component (typically the antigen component rather than the adjuvant component) is in dry form (*e.g.* in a lyophilised form), with the other component being in aqueous form. The two components can be mixed in order to reactivate the dry component and give an aqueous composition for administration to a patient. A lyophilised component will typically be located within a vial rather than a syringe. Dried components may include stabilizers such as lactose, sucrose or mannitol, as well as mixtures thereof *e.g.* lactose/sucrose mixtures, sucrose/mannitol mixtures, *etc.* One possible arrangement uses an aqueous adjuvant component in a pre-filled syringe and a lyophilised antigen component in a vial.

### Packaging of compositions or kit components

Suitable containers for compositions of the invention (or kit components) include vials, syringes (*e.g.* disposable syringes), nasal sprays, *etc.* These containers should be sterile.

Where a composition/component is located in a vial, the vial is preferably made of a glass or plastic material. The vial is preferably sterilized before the composition is added to it. To avoid problems with latex-sensitive patients, vials are preferably sealed with a latex-free stopper, and the absence of latex in all packaging material is preferred. The vial may include a single dose of vaccine, or it may include more than one dose (a 'multidose' vial) *e.g.* 10 doses. Preferred vials are made of colorless glass.

A vial can have a cap (*e.g.* a Luer lock) adapted such that a pre-filled syringe can be inserted into the cap, the contents of the syringe can be expelled into the vial (*e.g.* to reconstitute lyophilised material therein), and the contents of the vial can be removed back into the syringe. After removal of the syringe from the vial, a needle can then be attached and the composition can be administered to a patient. The cap is preferably located inside a seal or cover, such that the seal or cover has to be removed before the cap can be accessed. A vial may have a cap that permits aseptic removal of its contents, particularly for multidose vials.

Where a component is packaged into a syringe, the syringe may have a needle attached to it. If a needle is not attached, a separate needle may be supplied with the syringe for assembly and use. Such a needle may be sheathed. Safety needles are preferred. 1-inch 23-gauge, 1-inch 25-gauge and 5/8-inch 25-gauge needles are typical. Syringes may be provided with peel-off labels on which the lot number, influenza season and expiration date of the contents may be printed, to facilitate record keeping. The plunger in the syringe preferably has a stopper to prevent the plunger from being accidentally removed during aspiration. The syringes may have a latex rubber cap and/or plunger. Disposable syringes contain a single dose of vaccine. The syringe will generally have a tip cap to seal the tip prior to attachment of a needle, and the tip cap is preferably made of a butyl rubber. If the syringe and needle are packaged separately then the needle is preferably fitted with a butyl rubber shield. Preferred syringes are those marketed under the trade name "Tip-Lok"™.

Containers may be marked to show a half-dose volume *e.g.* to facilitate delivery to children. For instance, a syringe containing a 0.5ml dose may have a mark showing a 0.25ml volume.

Where a glass container (*e.g.* a syringe or a vial) is used, then it is preferred to use a container made from a borosilicate glass rather than from a soda lime glass.

A kit or composition may be packaged (*e.g.* in the same box) with a leaflet including details of the vaccine *e.g.* instructions for administration, details of the antigens within the vaccine, *etc.* The instructions may also contain warnings *e.g.* to keep a solution of adrenaline readily available in case of anaphylactic reaction following vaccination, *etc.* In some aspects of the disclosure, the leaflet will state that the vaccine includes matrix protein.

### Methods of treatment, and administration of the vaccine

Compositions of the disclosure are suitable for administration to human patients, and the disclosure provides a method of raising an immune response in a patient, comprising the step of administering a composition of the disclosure to the patient.

The disclosure also provides a kit or composition as described herein for use as a medicament.

The disclosure includes the use of (i) an influenza virus antigen preparation including haemagglutinin and matrix proteins, prepared from virus grown in cell culture, in the manufacture of a medicament for raising an immune response in a patient.

The immune response raised by these methods and uses will generally include an antibody response, preferably a protective antibody response. Methods for assessing antibody responses, neutralising capability and protection after influenza virus vaccination are well known in the art. Human studies have shown that antibody titers against hemagglutinin of human influenza virus are correlated with protection (a serum sample hemagglutination-inhibition titer of about 30-40 gives around 50% protection from infection by a homologous virus) [209]. Antibody responses are typically measured by hemagglutination inhibition, by microneutralisation, by single radial immunodiffusion (SRID), and/or by single radial hemolysis (SRH). These assay techniques are well known in the art.

Compositions of the disclosure can be administered in various ways. The most preferred immunisation route is by intramuscular injection (*e.g.* into the arm or leg), but other available routes include subcutaneous injection, intranasal [210-212], oral [213], intradermal [214,215], transcutaneous, transdermal [216], *etc.*

Vaccines prepared according to the invention may be used to treat both children and adults. Influenza vaccines are currently recommended for use in pediatric and adult immunisation, from the age of 6 months. Thus the patient may be less than 1 year old, 1-5 years old, 5-15 years old, 15-55 years old, or at least 55 years old. Preferred patients for receiving the vaccines are the elderly (*e.g.* ≥50 years old, ≥60 years old, and preferably ≥65 years), the young (*e.g.* ≤5 years old), hospitalised patients, healthcare workers, armed service and military personnel, pregnant women, the chronically ill, immunodeficient patients, patients who have taken an antiviral compound (*e.g.* an oseltamivir or zanamivir compound; see below) in the 7 days prior to receiving the vaccine, people with egg allergies and people travelling abroad. The vaccines are not suitable solely for these groups, however, and may be used more generally in a population. For pandemic strains, administration to all age groups is preferred.

Preferred compositions of the disclosure satisfy 1, 2 or 3 of the CPMP criteria for efficacy. In adults (18-60 years), these criteria are: (1) ≥70% seroprotection; (2) ≥40% seroconversion; and/or (3) a GMT increase of ≥2.5-fold. In elderly (>60 years), these criteria are: (1) ≥60% seroprotection; (2) ≥30% seroconversion; and/or (3) a GMT increase of ≥2-fold. These criteria are based on open label studies with at least 50 patients.

Treatment can be by a single dose schedule or a multiple dose schedule. Multiple doses may be used in a primary immunisation schedule and/or in a booster immunisation schedule. In a multiple dose schedule the various doses may be given by the same or different routes *e.g.* a parenteral prime and mucosal boost, a mucosal prime and parenteral boost, *etc.* Administration of more than one dose (typically two doses) is particularly useful in immunologically naive patients *e.g.* for people who have never received an influenza vaccine before, or for vaccinating against a new HA subtype (as in a pandemic outbreak). Multiple doses will typically be administered at least 1 week apart (*e.g.* about 2 weeks, about 3 weeks, about 4 weeks, about 6 weeks, about 8 weeks, about 10 weeks, about 12 weeks, about 16 weeks, *etc.*).

Vaccines produced by the invention may be administered to patients at substantially the same time as (*e.g.* during the same medical consultation or visit to a healthcare professional or vaccination centre) other vaccines *e.g.* at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated *H.influenzae* type b vaccine, an inactivated poliovirus vaccine, a hepatitis B virus vaccine, a meningococcal conjugate vaccine (such as a tetravalent A-C-W135-Y vaccine), a respiratory syncytial virus vaccine, a pneumococcal conjugate vaccine, *etc.* Administration at substantially the same time as a pneumococcal vaccine and/or a meningococcal vaccine is particularly useful in elderly patients.

Similarly, vaccines of the disclosure may be administered to patients at substantially the same time as (e.g. during the same medical consultation or visit to a healthcare professional) an antiviral compound, and in particular an antiviral compound active against influenza virus (*e.g.* oseltamivir and/or zanamivir). These antivirals include neuraminidase inhibitors, such as a (3R,4R,5S)-4-acetylamino-5-amino-3(1-ethylpropoxy)-1-cyclohexene-1-carboxylic acid or 5-(acetylamino)-4-[(aminoiminomethyl)-amino]-2,6-anhydro-3,4,5-trideoxy-D-glycero-D-galactonon-2-enonic acid, including esters thereof (*e.g.* the ethyl esters) and salts thereof (*e.g.* the phosphate salts). A preferred antiviral is (3R,4R,5S)-4-acetylamino-5-amino-3(1-ethylpropoxy)-1-cyclohexene-1-carboxylic acid, ethyl ester, phosphate (1:1), also known as oseltamivir phosphate (TAMIFLU™).

### Assays

To characterize an influenza vaccine, it may be useful to determine the amount of matrix protein that is present. Thus the disclosure includes assays for analyzing influenza vaccines, where a sample of the vaccine is analyzed to determine the presence of matrix protein. The assays are particularly useful for testing for fragments of M1 protein.

The influenza vaccine may include antigens from influenza A and/or influenza B viruses. The invention provides assays for analyzing vaccines comprising antigens from an influenza B virus, where a sample of the vaccine is analyzed to determine the presence of an influenza B virus matrix protein.

The influenza vaccine may include antigens from various HA subtypes. The disclosure includes assays for analyzing vaccines comprising antigens from an influenza A virus, where a sample of the vaccine is analyzed to determine the presence of an influenza A virus matrix protein, and where the influenza A virus has a hemagglutinin subtype selected from: H2, H4, H5, H6, H7, H8, H9, H10, H11, H12, H13, H14, H15 or H16.

The influenza vaccine may include antigens grown on cell culture. The disclosure includes assays for analyzing vaccines grown on cell culture, where a sample of the vaccine is analyzed to determine the presence of an influenza virus matrix protein.

The influenza vaccine may include an adjuvant. The disclosure includes assays for analyzing adjuvanted influenza vaccines, where a sample of the vaccine is analyzed to determine the presence of an influenza virus matrix protein. The disclosure includes assays for analyzing unadjuvanted influenza vaccines, where a sample of the unadjuvanted vaccine is analyzed to determine the presence of an influenza virus matrix protein, and wherein an adjuvant is then added to the vaccine.

These assays will typically be immunoassays, such as a western blot or ELISA. An immunoassay may use polyclonal or monoclonal antibody. Where a monoclonal antibody is used, in some embodiments it is not a murine antibody, such as a murine IgG1 antibody. Antibodies that recognize M1 fragments can be used in the assays, including those that recognize the M1 fragments disclosed herein *e.g.* antibodies that recognize fragments with a molecular weight of 10kDa or less (*e.g.* ≤5kDa), that recognize fragments lacking the N terminal methionine, that recognize fragments with N terminal sequence of SEQ ID NO: 15, that recognize SEQ ID NO: 28 and/or 29, that recognize fragments including SEQ ID NO: 30, that recognize fragments with a covalently-modified N-terminal residue, *etc.*

The assays are particularly useful for detecting fragments of M1 protein, such fragments with a molecular weight of 10kDa or less (*e.g.* ≤5kDa), and/or the fragments disclosed herein (*e.g.* lacking the N terminal methionine of the full M1 sequence). Preferred assays can distinguish between the presence of full-length M1 protein and fragments of M1 protein, and may also distinguish between different fragments.

The assays may be qualitative, semi-quantitative or quantitative.

The disclosure includes vaccines that have been assayed in this way.

### Further aspects of the invention

The disclosure includes an immunogenic composition comprising (i) influenza virus haemagglutinin and matrix proteins, and (ii) an adjuvant. The influenza virus proteins may be prepared from virus grown in cell culture or grown in egg. As described above, the composition may also include further components *e.g.* influenza virus neuraminidase protein, pharmaceutical carriers/excipients, *etc.*

The disclosure also provides a method for preparing an immunogenic composition comprising the steps of: (i) growing influenza virus; (ii) preparing an antigen composition from the viruses grown in step (i), wherein the antigen composition comprises haemagglutinin and matrix proteins; and (iii) combining the antigen composition with an adjuvant, to give the immunogenic composition.

The disclosure includes an immunogenic composition comprising (i) influenza virus haemagglutinin and matrix proteins, wherein the haemagglutinin has a subtype selected from: H2, H4, H5, H6, H7, H8, H9, H10, H11, H12, H13, H14, H15 or H16.

The disclosure also provides a method for preparing an immunogenic composition comprising the steps of: (i) growing influenza virus; (ii) preparing an antigen composition from the viruses grown in step (i), wherein the antigen composition comprises haemagglutinin and matrix proteins, and wherein the haemagglutinin has a subtype selected from: H2, H4, H5, H6, H7, H8, H9, H10, H11, H12, H13, H14, H15 or H16; and (iii) combining the antigen composition with an adjuvant, to give the immunogenic composition.

The disclosure includes an immunogenic composition comprising (i) influenza virus haemagglutinin and matrix proteins, wherein the concentration of haemagglutinin in the composition is 29µg/ml or lower (e.g. ≤28µg/ml, ≤27µg/ml, ≤26µg/ml, ≤25µg/ml, ≤24µg/ml, ≤23µg/ml, ≤22µg/ml, ≤21µg/ml, ≤20µg/ml, ≤19µg/ml, ≤18µg/ml, ≤17µg/ml, ≤16µg/ml, ≤15µg/ml, ≤14µg/ml, ≤13µg/ml, ≤12µg/ml, ≤11µg/ml, ≤10µg/ml, ≤9µg/ml, ≤8µg/ml, ≤7µg/ml, ≤6µg/ml, ≤5µg/ml, ≤4µg/ml, ≤3µg/ml, ≤2µg/ml). The composition may include haemagglutinin from more than one strain of influenza virus, in which case the said concentration is per strain (*i.e.* ≤29µg/ml per strain).

The disclosure includes an immunogenic composition comprising (i) influenza virus haemagglutinin and matrix proteins, wherein the concentration of haemagglutinin in the composition is 31µ/ml or higher (*e.g.* ≥32µg/ml, ≥33µg/ml, ≥34µg/ml, ≥35µg/ml, ≥40µg/ml, ≥45µg/ml, ≥50µg/ml, ≥55µg/ml, ≥60µg/ml, ≥70µg/ml, ≥80µg/ml, ≥90µg/ml, ≥100µg/ml, *etc,* but typically <200µg). The composition may include haemagglutinin from more than one strain of influenza virus, in which case the said concentration is per strain (*i.e*. ≥31µg/ml per strain).

The disclosure includes an immunogenic composition comprising an influenza virus M2 matrix protein, but being substantially free from an influenza virus matrix protein M1 as defined above. M2-containing vaccines are currently being developed [217]. M2 is naturally encoded by a viral segment that also encodes M1. In a recombinant expression system, M1 protein might thus be expressed as a side-product. According to the disclosure, either this side-expression can be avoided, or else the M1 matrix proteins can be removed from the M2-containing composition. The M2 protein may be a full-length protein or may, for instance, be a M2 fragment, such as the M2 extracellular domain (known in the art as 'M2e'). The M2 protein may be conjugated to another antigen *e.g.* to a hepatitis B virus antigen. The vaccine may also be free from HA and/or NA.

### General

The term "comprising" encompasses "including" as well as "consisting" *e.g.* a composition "comprising" X may consist exclusively of X or may include something additional *e.g.* X + Y.

The word "substantially" does not exclude "completely" e.g. a composition which is "substantially free" from Y may be completely free from Y. Where necessary, the word "substantially" may be omitted from the definition of the invention.

The term "about" in relation to a numerical value *x* means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

Where a cell substrate is used for reassortment or reverse genetics procedures, it is preferably one that has been approved for use in human vaccine production *e.g.* as in Ph Eur general chapter 5.2.3.

Identity between polypeptide sequences is preferably determined by the Smith-Waterman homology search algorithm as implemented in the MPSRCH program (Oxford Molecular), using an affine gap search with parameters *gap open penalty*=*12* and *gap extension penalty*=*1.*

### BRIEF DESCRIPTION OF THE DRAWINGS

Figure 1 is an SDS-PAGE of various vaccine preparations. The 'M' lanes are molecular weight markers (kDa). Lane 1 is the MDCK-grown vaccine. Lanes 2-6 are existing commercial vaccines. The arrows show (a) HA₁ (b) HA₂ (c) the M1 fragment of the invention.
Figure 2 shows the results of western blot analysis of fractionated influenza virions using antisera from rabbits immunized with the ∼5kDa matrix protein fragment. The left-hand panel is a blot using pre-immune sera, and the right-hand panel uses post-immune sera.

### MODES FOR CARRYING OUT THE INVENTION

While working on a purified surface antigens vaccine for influenza virus, where virions were grown on MDCK cells, it was observed that a relatively large amount of low MW polypeptide could be detected by SDS PAGE after splitting of influenza A virus with CTAB. This low MW polypeptide was also present during further antigen purification, and was present in the final preparation of surface antigens. To investigate this polypeptide, a buffer system was used that allows the identification of polypeptides as small as 2 kDa (NuPAGE™ Novex Bis-Tris Gels from Invitrogen). Using this system, a polypeptide band with an apparent MW of ∼5 kDa was identified. This polypeptide band was not seen in the current INFLEXAL V™, INFLUSPLIT™, MUTAGRIP™, VAXIGRIP™, BEGRIVAC™, FLUARIX™, INFLUVAC™ or FLUVIRIN™ vaccines, all of which are prepared from egg-grown virions. Surprisingly, the polypeptide band was detected in some batches of AGRIPPAL™, but its existence or presence was not previously recognised.

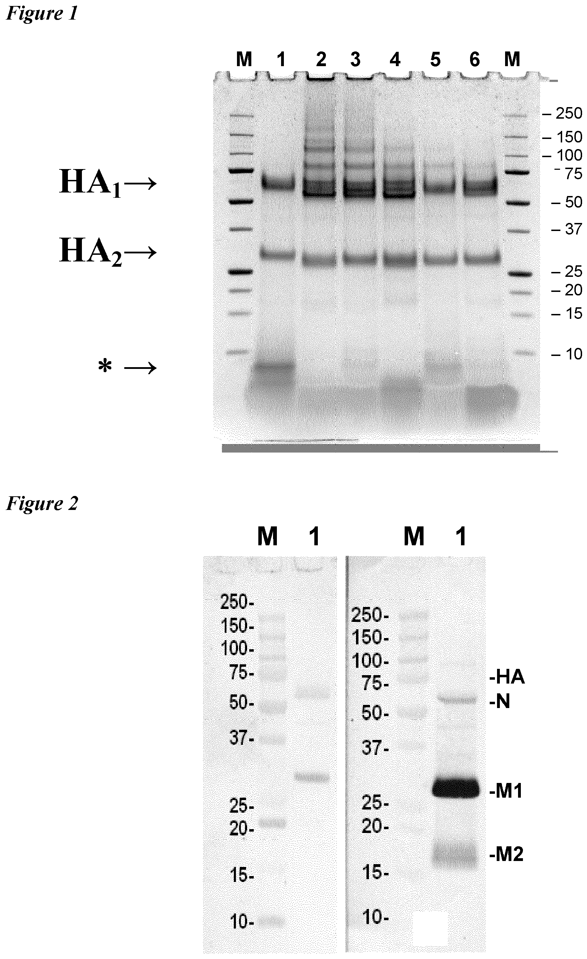

Immunization of rabbits with the ∼5 kDa band induced antibodies which were able to detect the native M1 protein. Figure 2 shows the results of western blot analysis of fractionated influenza virions using antisera from the rabbits, with strong reactivity to virion M1 protein. Thus, a polypeptide in the ∼5 kDa band carries epitopes that are immunogenic and can cause the production of antibodies which bind to the native corresponding M1 protein.

N-terminal amino acid sequence analysis of the ∼5 kDa bands derived from two different viruses (a H1N1 virus and a H3N2 virus) revealed a peptide with N-terminal sequence of EISLSYSAGALA (SEQ ID NO: 18). This amino acid sequence is a fragment of the influenza virus M1 protein identical with the amino acid positions 114 to 125 of SEQ ID NO: 2.

Further investigation using MS analysis of tryptic fragments revealed a more abundant fragment with N-terminal amino acid sequence SEQ ID NO: 28, which is lacking the N-terminal methionine of SEQ ID NO: 2:

The C-terminus of this fragment may have an additional Arg residue (*i.e*. SLL...QRR, SEQ ID NO: 29). The N-terminal serine may be covalently modified e.g. acetylated. The N-terminal 12-mer of this fragment (SLLTEVETYVLS; SEQ ID NO: 30) is well conserved between strains.

Compositions of the disclosure may contain both such fragments, with the fragment from near the N-terminal (*e.g.* SEQ ID NO: 28) being more abundant.

### REFERENCES

*[1]* Vaccines. (eds. Plotkin & Orenstein). 4th edition, 2004, ISBN: 0-7216-9688-0.
[2] Chaloupka et al. (1996) Eur J Clin Microbiol Infect Dis 15:121-7.
[3] WO96/37624.
[4] WO98/46262.
[5] WO02/28422.
[6] WO02/067983.
[7] WO02/074336.
[8] WO01/21151.
[9] WO02/097072.
[10] WO2005/113756.
[11] Huckriede et al. (2003) Methods Enzymol 373:74-91.
[12] World Health Organisation (2005) Emerging Infectious Diseases 11(10):1515-21.
[13] Herlocher et al. (2004) J Infect Dis 190(9):1627-30.
[14] Le et al. (2005) Nature 437(7062):1108.
[15] Hoffmann et al. (2002) Vaccine 20:3165-3170.
[16] Subbarao et al. (2003) Virology 305:192-200.
[17] Liu et al. (2003) Virology 314:580-590.
[18] Ozaki et al. (2004) J. Virol. 78:1851-1857.
[19] Webby et al. (2004) Lancet 363:1099-1103.
[20] WO00/60050.
[21] WO01/04333.
[22] US patent 6649372.
[23] Neumann et al. (2005) Proc Natl Acad Sci USA 102:16825-9.
[24] WO2006/067211.
[25] WO01/83794.
[26] Hoffmann et al. (2000) Virology 267(2):310-7.
[27] WO97/37000.
[28] Brands et al. (1999) Dev Biol Stand 98:93-100.
[29] Halperin et al. (2002) Vaccine 20:1240-7.
[30] Tree et al. (2001) Vaccine 19:3444-50.
[31] Kistner et al. (1998) Vaccine 16:960-8.
[32] Kistner et al. (1999) Dev Biol Stand 98:101-110.
[33] Bruhl et al. (2000) Vaccine 19:1149-58.
[34] Pau et al. (2001) Vaccine 19:2716-21.
[35] *http:*//*www.atcc.org*/
[36] *http:*//*locus.umdnj.edu*/
[37] WO03/076601.
[38] WO2005/042728.
[39] WO03/043415.
[40] WO01/85938.
[41] WO2006/108846.
[42] EP-A-1260581 (WO01/64846).
[43] WO2006/071563.
[44] WO2005/113758.
[45] WO2006/027698.
[46] WO97/37000
[47] WO97/37001.
[48] Treanor et al. (1996) J Infect Dis 173:1467-70.
[49] Keitel et al. (1996) Clin Diagn Lab Immunol 3:507-10.
[50] Geysen et al. (1984) PNAS USA 81:3998-4002.
[51] Carter (1994) Methods Mol Biol 36:207-23.
[52] Jameson, BA et al. 1988, CABIOS 4(1):181-186.
[53] Raddrizzani & Hammer (2000) Brief Bioinform 1(2):179-89.
[54] De Lalla et al. (1999) J. Immunol. 163:1725-29.
[55] Brusic et al. (1998) Bioinformatics 14(2):121-30
[56] Meister et al. (1995) Vaccine 13(6):581-91.
[57] Roberts et al. (1996) AIDS Res Hum Retroviruses 12(7):593-610.
[58] Maksyutov & Zagrebelnaya (1993) Comput Appl Biosci 9(3):291-7.
[59] Feller & de la Cruz (1991) Nature 349(6311):720-1.
[60] Hopp (1993) Peptide Research 6:183-190.
[61] Welling et al. (1985) FEBS Lett. 188:215-218.
[62] Davenport et al. (1995) Immunogenetics 42:392-297.
[63] Textbook of Influenza (Karl Nicholson, Robert Webster, Alan Hay and Nancy Cox, eds).
[64] Gotch et al. (1988) J Exp Med 168(6):2045-57.
[65] Gianfrani et al. (2000) Human Immunol 61:438-452.
[66] Vitiello et al. (1996) J Immunol 157(12):5555-62.
[67] Dong et al. (1996) Eur J Immunol 26(2):335-339.
[68] Andersen et al. (1999) Tissue Antigens 54(2):185-190.
[69] Adler et al. (1994) FEBS Lett 352(2):167-170.
[70] Rothbard et al. (1988) Cell 52(4):515-523.
[71] Evans et al. (1999) J Immunol 162(7):3970-3977.
[72] Elster et al. (1994) J Gen Virol 75:37-42.
[73] Macken et al. (2001) in Options for the Control of Inluenza IV (eds. Osterhaus *et al*.).
[74] Spackman et al. (2005) Virus Res 114(1-2):89-100.
[75] Webster & Hinshaw (1977) Infect Immun 17:561-6.
[76] Hui et al. (2003) J Gen Virol 84:3105-13
[77] Elster et al. (1997) J Gen Virol 78:1589-96.
[78] Lundblad (2001) Biotechnology and Applied Biochemistry 34:195-197.
*[79]* Guidance for Industry: Bioanalytical Method Validation. U.S. Department of Health and Human Services Food and Drug Administration Center for Drug Evaluation and Research (CDER) Center for Veterinary Medicine (CVM). May 2001.
[80] Ji et al. (2002) Biotechniques. 32:1162-7.
[81] Briggs (1991) J Parenter Sci Technol. 45:7-12.
[82] Lahijani et al. (1998) Hum Gene Ther. 9:1173-80.
[83] Lokteffet al. (2001) Biologicals. 29:123-32.
[84] EP-B-0870508.
[85] US 5948410.
[86] WO2007/052163.
[87] US patent 6,372,223.
[88] WO00/15251.
[89] WO01/22992.
[90] Hehme et at. (2004) Virus Res. 103(1-2):163-71.
[91] US patent 6355271.
[92] WO00/23105.
[93] US 5,057,540.
[94] WO96/33739.
[95] EP-A-0109942.
[96] WO96/11711.
[97] WO00/07621.
[98] Barr et al. (1998) Advanced Drug Delivery Reviews 32:247-271.
[99] Sjolanderet et al. (1998) Advanced Drug Delivery Reviews 32:321-338.
[100] Pizza et al. (2000) Int J Med Microbiol 290:455-461.
[101] WO95/17211.
[102] WO98/42375.
[103] Singh et al] (2001) J Cont Release 70:267-276.
[104] WO99/27960.
[105] US 6,090,406
[106] US 5,916,588
[107] EP-A-0626169.
[108] WO99/52549.
[109] WO01/21207.
[110] WO01/21152.
[111] WO02/072012.
[112] Dyakonova et al. (2004) Int Immunopharmacol 4(13):1615-23.
[113] FR-2859633.
[114] Signorelli & Hadden (2003) Int Immunopharmacol 3(8):1177-86.
[115] WO2004/064715.
[116] De Libero et al, Nature Reviews Immunology, 2005, 5: 485-496
[117] US patent 5,936,076.
[118] Oki et al, J. Clin. Investig., 113: 1631-1640
[119] US2005/0192248
[120] Yang et al, Angew. Chem. Int. Ed., 2004, 43: 3818-3822
[121] WO2005/102049
[122] Goff et al, J. Am. Chem., Soc., 2004, 126: 13602-13603
[123] WO03/105769
[124] Cooper (1995) Pharm Biotechnol 6:559-80.
[125] WO90/14837.
[126] Podda & Del Giudice (2003) Expert Rev Vaccines 2:197-203.
[127] Podda (2001) Vaccine 19: 2673-2680.
[128] Vaccine Design: The Subunit and Adjuvant Approach (eds. Powell & Newman) Plenum Press 1995 (ISBN 0-306-44867-X).
[129] Vaccine Adjuvants: Preparation Methods and Research Protocols (Volume 42 of Methods in Molecular Medicine series). ISBN: 1-59259-083-7. Ed. O'Hagan.
[130] Allison & Byars (1992) Res Immunol 143:519-25.
[131] Hariharan et al. (1995) Cancer Res 55:3486-9.
[132] WO95/11700.
[133] US patent 6,080,725.
[134] WO2005/097181.
[135] Han et al. (2005) Impact of Vitamin E on Immune Function and Infectious Diseases in the Aged at Nutrition, Immune functions and Health EuroConference, Paris, 9-10 June 2005.
[136] US- 6630161.
[137] Hayden et al. (1998) J Clin Invest 101(3):643-9.
[138] Tassignon et al. (2005) J Immunol Meth 305:188-98.
[139] Myers et al. (1990) pages 145-156 of Cellular and molecular aspects of endotoxin reactions*.*
[140] Ulrich (2000) Chapter 16 (pages 273-282) of reference 129.
[141] Johnson et al. (1999) J Med Chem 42:4640-9.
[142] Baldrick et al. (2002) Regulatory Toxicol Pharmacol 35:398-413.
[143] US 4,680,338.
[144] US 4,988,815.
[145] WO92/15582.
[146] Stanley (2002) Clin Exp Dermatol 27:571-577.
[147] Wu et al. (2004) Antiviral Res. 64(2):79-83.
[148] Vasilakos et al. (2000) Cell Immunol. 204(1):64-74.
[149] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.
[150] Jones (2003) Curr Opin Investig Drugs 4:214-218.
[151] WO2004/060308.
[152] WO2004/064759.
[153] US 6,924,271.
[154] US2005/0070556.
[155] US 5,658,731.
[156] US patent 5,011,828.
[157] WO2004/87153.
[158] US 6,605,617.
[159] WO02/18383.
[160] WO2004/018455.
[161] WO03/082272.
[162] WO2006/002422.
[163] Johnson et al. (1999) Bioorg Med Chem Lett 9:2273-2278.
[164] Evans et al. (2003) Expert Rev Vaccines 2:219-229.
[165] Andrianov et al. (1998) Biomaterials 19:109-115.
[166] Payne et al. (1998) Adv Drug Delivery Review 31:185-196.
[167] Thompson et al. (2003) Methods in Molecular Medicine 94:255-266.
[168] Kandimalla et al. (2003) Nucleic Acids Research 31:2393-2400.
[169] WO02/26757.
[170] WO99/62923.
[171] Krieg (2003) Nature Medicine 9:831-835.
[172] McCluskie et al. (2002) FEMS Immunology and Medical Microbiology 32:179-185.
[173] WO98/40100.
[174] US patent 6,207,646.
[175] US patent 6,239,116.
[176] US patent 6,429,199.
[177] Kandimalla et al. (2003) Biochemical Society Transactions 31 (part 3):654-658.
[178] Blackwell et al. (2003) J Immunol 170:4061-4068.
[179] Krieg (2002) Trends Immunol 23:64-65.
[180] WO01/95935.
[181] Kandimalla et al. (2003) BBRC 306:948-953.
[182] Bhagat et al. (2003) BBRC 300:853-861.
[183] WO03/035836.
[184] WO01/22972.
[185] Thompson et al. (2005) J Leukoc Biol 78: 'The low-toxicity versions of LPS, MPL® adjuvant and RC529, are efficient adjuvants for CD4+ T cells'.
[186] UK patent application GB-A-2220211.
[187] WO 94/21292.
[188] WO94/00153.
[189] WO95/17210.
[190] WO96/26741.
[191] WO93/19780.
[192] WO03/011223.
[193] Meraldi et al. (2003) Vaccine 21:2485-2491.
[194] Pajak et al. (2003) Vaccine 21:836-842.
[195] US-6586409.
[196] Wong et al. (2003) J Clin Pharmacol 43(7):735-42.
[197] US2005/0215517.
[198] Gennaro (2000) Remington: The Science and Practice of Pharmacy. 20th edition, ISBN: 0683306472.
[199] Banzhoff (2000) Immunology Letters 71:91-96.
[200] Nony et al. (2001) Vaccine 27:3645-51.
[201] WO2005/089837.
[202] US patent 6,692,468.
[203] WO00/07647.
[204] WO99/17820.
[205] US patent 5,971,953.
[206] US patent 4,060,082.
[207] EP-A-0520618.
[208] WO98/01174.
[209] Potter & Oxford (1979) Br Med Bull 35: 69-75.
[210] Greenbaum et al. (2004) Vaccine 22:2566-77.
[211] Zurbriggen et al. (2003) Expert Rev Vaccines 2:295-304.
[212] Piascik (2003) J Am Pharm Assoc (Wash DC). 43:728-30.
[213] Mann et al. (2004) Vaccine 22:2425-9.
[214] Halperin et al. (1979) Am J Public Health 69:1247-50.
[215] Herbert et al. (1979) J Infect Dis 140:234-8.
[216] Chen et al. (2003) Vaccine 21:2830-6.
[217] De Filette et al. (2005) Virology 337(1):149-61.

## Claims

1. A method for preparing an immunogenic composition comprising the steps of:
(i) growing influenza virus in cell culture wherein trypsin is added to allow viral release;
(ii) preparing an antigen composition from the viruses grown in step (i), wherein the antigen composition comprises haemagglutinin and matrix proteins not as a whole virion which form a stable complex; and
(iii) combining the antigen composition with a pharmaceutical carrier, to give the immunogenic composition
wherein the matrix protein has an amino acid sequence which is a fragment of a full-length M1 matrix protein amino acid sequence.

2. The method of claim 1, wherein the influenza viruses are grown in MDCK cells, for example MDCK 33016 (DSM ACC 2219).

3. The method of claim 1 or claim 2, wherein the influenza viruses are grown in a cell line which is adapted for growth in suspension.

4. The method of any preceding claim, wherein the antigen composition in step (ii) is prepared by splitting the influenza viruses with cetyl trimethyl ammonium bromide.

5. The method of any preceding claim, wherein the matrix protein comprises a sequence of 20 amino acids that has at least 80% identity to SEQ ID NO: 2 and/or wherein the matrix protein comprises a T cell epitope from influenza virus M1 protein.

6. The method of any preceding claim, wherein the matrix protein comprises one or the following amino acid sequences: SEQ ID NO: 1; SEQ ID NO: 21; SEQ ID NO: 22; SEQ ID NO: 23; SEQ ID NO: 24; SEQ ID NO: 25; SEQ ID NO: 26; SEQ ID NO: 27.

7. The method of any preceding claim, wherein the matrix protein lacks the N-terminal methionine of the natural M1 sequence.

8. The method of claim 7, wherein the matrix protein has a N-terminal sequence SLLTEVETYVLS (SEQ ID NO: 30), for example wherein the N-terminal serine of SEQ ID NO: 30 is covalently modified e.g. acetylated.

9. The method of any one of claims 1 to 7, wherein the matrix protein has a N-terminal sequence EISLSYSAGALA (SEQ ID NO: 18).

10. The method of any preceding claim, wherein the composition comprises: (i) a first matrix protein having a N-terminal sequence SLLTEVETYVLS (SEQ ID NO: 30); and (ii) a second matrix protein having a N-terminal sequence EISLSYSAGALA (SEQ ID NO: 18).

11. The method of any preceding claim, wherein matrix protein is present at a concentration between 1µg/ml and 15 µg/ml.

12. The method of any preceding claim, wherein the immunogenic composition comprises split influenza virus or purified influenza surface antigens.

13. The method of any preceding claim, wherein the haemagglutinin is from a H1, H2, H3, H5, H7 or H9 influenza A virus subtype.

14. The method of any preceding claim, wherein the influenza virus proteins is prepared from an influenza virus grown on a culture of a host cell and the composition contains less than 10ng of cellular DNA from the host cell.

15. The method of any preceding claim, wherein the composition contains between 0.1 and 20µg of haemagglutinin per viral strain.

16. The method of any preceding claim, wherein the composition comprises antigens from 1, 2, 3, 4 or more influenza strains, wherein the strains are influenza A virus and/or influenza B virus.

17. The method of any preceding claim, wherein the antigens are prepared from virions that are inactivated.

18. The method of any preceding claim, wherein the composition includes an adjuvant, for example an oil-in-water emulsion, or one or more aluminium salts.

## Patentansprüche

1. Verfahren zur Herstellung einer immunogenen Zusammensetzung, umfassend die Schritte:
(i) Kultivieren von Influenzavirus in Zellkultur, wobei Trypsin zugegeben wird, um die Virusfreisetzung zu ermöglichen;
(ii) Herstellen einer Antigenzusammensetzung aus den in Schritt (i) kultivierten Viren, wobei die Antigenzusammensetzung Hämagglutinin und Matrixproteine nicht in Form eines intakten Virions umfasst, die einen stabilen Komplex bilden; und
(iii) Kombinieren der Antigenzusammensetzung mit einem pharmazeutischen Träger unter Erhalt der immunogenen Zusammensetzung,
wobei das Matrixprotein eine Aminosäuresequenz aufweist, bei der es sich um ein Fragment einer Volllängen-M1-Matrixprotein-Aminosäuresequenz handelt.

2. Verfahren nach Anspruch 1, wobei die Influenzaviren in MDCK-Zellen kultiviert werden, z. B. MDCK 33016 (DSM ACC 2219).

3. Verfahren nach Anspruch 1 oder Anspruch 2, wobei die Influenzaviren in einer Zelllinie kultiviert werden, die an Wachstum in Suspension angepasst ist.

4. Verfahren nach einem vorhergehenden Anspruch, wobei die Antigenzusammensetzung in Schritt (ii) durch Spalten der Influenzaviren mit Cetyltrimethylammoniumbromid hergestellt wird.

5. Verfahren nach einem vorhergehenden Anspruch, wobei das Matrixprotein eine Sequenz von 20 Aminosäuren umfasst, die eine Identität von wenigstens 80% mit SEQ ID NO: 2 aufweist und/oder wobei das Matrixprotein ein T-Zell-Epitop aus Influenzavirus-M1-Protein umfasst.

6. Verfahren nach einem vorhergehenden Anspruch, wobei das Matrixprotein eine oder die folgenden Aminosäuresequenzen umfasst: SEQ ID NO: 1; SEQ ID NO: 21; SEQ ID NO: 22; SEQ ID NO: 23; SEQ ID NO: 24; SEQ ID NO: 25; SEQ ID NO: 26; SEQ ID NO: 27.

7. Verfahren nach einem vorhergehenden Anspruch, wobei dem Matrixprotein das N-terminale Methionin der natürlichen M1-Sequenz fehlt.

8. Verfahren nach Anspruch 7, wobei das Matrixprotein eine N-terminale Sequenz SLLTEVETYVLS (SEQ ID NO: 30) aufweist, zum Beispiel wobei das N-terminale Serin von SEQ ID NO: 30 kovalent modifiziert, z.B. acetyliert, ist.

9. Verfahren nach einem der Ansprüche 1 bis 7, wobei das Matrixprotein eine N-terminale Sequenz EISLSYSAGALA (SEQ ID NO: 18) aufweist.

10. Verfahren nach einem vorhergehenden Anspruch, wobei die Zusammensetzung (i) ein erstes Matrixprotein mit einer N-terminalen Sequenz SLLTEVETYVLS (SEQ ID NO: 30); und (ii) ein zweites Matrixprotein mit einer N-terminalen Sequenz EISLSYSAGALA (SEQ ID NO: 18) umfasst.

11. Verfahren nach einem vorhergehenden Anspruch, wobei Matrixprotein in einer Konzentration zwischen 1 µg/ml und 15 µg/ml vorliegt.

12. Verfahren nach einem vorhergehenden Anspruch, wobei die immunogene Zusammensetzung gespaltenes Influenzavirus oder aufgereinigte Influenza-Oberflächenantigene umfasst.

13. Verfahren nach einem vorhergehenden Anspruch, wobei das Hämagglutinin aus einem H1-, H2-, H3-, H5-, H7- oder H9-Influenza-A-Virus-Subtyp stammt.

14. Verfahren nach einem vorhergehenden Anspruch, wobei die Influenzavirusproteine aus einem auf einer Kultur einer Wirtszelle kultivierten Influenzavirus präpariert werden und die Zusammensetzung weniger als 10 ng zelluläre DNA aus der Wirtszelle enthält.

15. Verfahren nach einem vorhergehenden Anspruch, wobei die Zusammensetzung zwischen 0,1 und 20 µg Hämagglutinin pro Virusstamm enthält.

16. Verfahren nach einem vorhergehenden Anspruch, wobei die Zusammensetzung Antigene aus 1, 2, 3, 4 oder mehr Influenzastämmen umfasst, wobei es sich bei den Stämmen um Influenza-A-Virus und/oder Influenza-B-Virus handelt.

17. Verfahren nach einem vorhergehenden Anspruch, wobei die Antigene aus Virionen präpariert werden, die inaktiviert sind.

18. Verfahren nach einem vorhergehenden Anspruch, wobei die Zusammensetzung ein Adjuvans enthält, zum Beispiel eine Öl-in-Wasser-Emulsion oder ein oder mehrere Aluminiumsalze.

## Revendications

1. Procédé pour la préparation d'une composition immunogène comprenant les étapes de
(i) croissance d'un virus de la grippe dans une culture cellulaire, dans laquelle de la trypsine est ajoutée pour permettre une libération virale;
(ii) préparation d'une composition d'antigène à partir des virus crus dans l'étape (i), dans laquelle la composition d'antigène comprend de l'hémagglutinine et des protéines matricielles pas en un virion entier qui forment un complexe stable ; et
(iii) combinaison de la composition d'antigène avec un support pharmaceutique, pour donner la composition immunogène
dans lequel la protéine matricielle possède une séquence d'acides aminés qui est un fragment d'une séquence d'acides aminés de protéine matricielle M1 de pleine longueur.

2. Procédé selon la revendication 1, dans lequel les virus de la grippe sont crus dans des cellules MCDK, par exemple MCDK 33 016 (DSM ACC 2119).

3. Procédé selon la revendication 1 ou la revendication 2, dans lequel les virus de la grippe sont crus dans une lignée cellulaire qui est adaptée pour une croissance en suspension.

4. Procédé selon l'une quelconque des revendications précédentes, dans lequel la composition d'antigène dans l'étape (ii) est préparée par fractionnement des virus de la grippe avec du bromure de cétyltriméthylammonium.

5. Procédé selon l'une quelconque des revendications précédentes, dans lequel la protéine de matrice comprend une séquence de 20 acides aminés qui a au moins 80 % d'identité avec SEQ ID NO : 2 et/ou dans lequel la protéine matricielle comprend un épitope de cellules T de protéine M1 du virus de la grippe.

6. Procédé selon l'une quelconque des revendications précédentes, dans lequel la protéine de matrice comprend une des séquences d'acides aminés suivantes : SEQ ID NO : 1; SEQ ID NO : 21; SEQ ID NO : 22; SEQ ID NO : 23; SEQ ID NO : 24; SEQ ID NO : 25; SEQ ID NO 26; SEQ ID NO : 27.

7. Procédé selon l'une quelconque des revendications précédentes, dans lequel la protéine de matrice est dépourvue de la méthionine N-terminale de la séquence M1 naturelle.

8. Procédé selon la revendication 7, dans lequel la protéine de matrice a une séquence N-terminale SLLTEVETYVLS (SEQ ID NO : 30), par exemple dans lequel la sérine N-terminale de SEQ ID NO : 30 est modifiée de manière covalente, par exemple, acétylée.

9. Procédé selon l'une quelconque des revendications 1 à 7, dans lequel la protéine de matrice a une séquence N-terminale EISLSYSAGALA (SEQ ID NO : 18).

10. Procédé selon l'une quelconque des revendications précédentes, dans lequel la composition comprend : (i) une première protéine de matrice ayant une séquence N-terminale SLLTEVETYVLS (SEQ ID NO : 30) ; et (ii) une seconde protéine de matrice ayant une séquence N-terminale EISLSYSAGALA (SEQ ID NO : 18).

11. Procédé selon l'une quelconque des revendications précédentes, dans lequel la protéine de matrice est présente à une concentration entre 1 µg/mL et 15 µg/mL.

12. Procédé selon l'une quelconque des revendications précédentes, dans lequel la composition immunogène comprend un virus de la grippe fractionné ou des antigènes de surface de la grippe purifiés

13. Procédé selon l'une quelconque des revendications précédentes, dans lequel l'hémagglutinine provient d'un sous-type de virus de la grippe A H1, H2, H3, H5, H7 ou H9.

14. Procédé selon l'une quelconque des revendications précédentes, dans lequel les protéines de virus de la grippe sont préparées à partir d'un virus de la grippe crû sur une culture d'une cellule hôte et la composition contient moins de 10 ng d'ADN cellulaire de la cellule hôte.

15. Procédé selon l'une quelconque des revendications précédentes, dans lequel la composition contient entre 0,1 et 20 µg d'hémagglutinine par souche virale.

16. Procédé selon l'une quelconque des revendications précédentes, dans lequel la composition comprend des antigènes de 1, 2, 3, 4 souches grippales ou plus, dans lequel les souches sont un virus de la grippe A et/ou un virus de la grippe B.

17. Procédé selon l'une quelconque des revendications précédentes, dans lequel les antigènes sont préparés à partir de virions qui sont inactivés.

18. Procédé selon l'une quelconque des revendications précédentes, dans lequel la composition inclut un adjuvant, par exemple une émulsion huile dans l'eau, ou un ou plusieurs sels d'aluminium.


### SEQUENCE LISTING

<110> Novartis Vaccines and Diagnostics GmbH
<120> INFLUENZA VACCINES CONTAINING HEMAGGLUTININ AND MATRIX PROTEINS
<130> P057212EP
<140> EP .....
   <141> 2007-01-26
<150> PCT/IB2007/001150
   <151> 2007-01-26
<150> GB-0601733.8
   <151> 2006-01-27
<150> GB-0620175.0
   <151> 2006-10-11
<160> 30
<170> SeqWin99, version 1.02
<210> 1
   <211> 9
   <212> PRT
   <213> Influenza virus
<220>
   <221> misc_feature
   <222> 5
   <223> Xaa is Ala or Thr
<400> 1
   Leu Ser Tyr Ser Xaa Gly Ala Leu Ala
   1               5
<210> 2
   <211> 252
   <212> PRT
   <213> Influenza virus
<400> 2
   Met Ser Leu Leu Thr Glu Val Glu Thr Tyr Val Leu Ser Ile Ile Pro
   1               5                   10                  15
   Ser Gly Pro Leu Lys Ala Glu Ile Ala Gln Arg Leu Glu Asp Val Phe
               20                  25                  30
   Ala Gly Lys Asn Thr Asp Leu Glu Val Leu Met Glu Trp Leu Lys Thr
           35                  40                  45
   Arg Pro Ile Leu Ser Pro Leu Thr Lys Gly Ile Leu Gly Phe Val Phe
       50                  55                  60
   Thr Leu Thr Val Pro Ser Glu Arg Gly Leu Gln Arg Arg Arg Phe Val
   65                  70                  75                  80
   Gln Asn Ala Leu Asn Gly Asn Gly Asp Pro Asn Asn Met Asp Lys Ala
                   85                  90                  95
   Val Lys Leu Tyr Arg Lys Leu Lys Arg Glu Ile Thr Phe His Gly Ala
               100                 105                 110
   Lys Glu Ile Ser Leu Ser Tyr Ser Ala Gly Ala Leu Ala Ser Cys Met
           115                 120                 125
   Gly Leu Ile Tyr Asn Arg Met Gly Ala Val Thr Thr Glu Val Ala Phe
       130                 135                 140
   Gly Leu Val Cys Ala Thr Cys Glu Gln Ile Ala Asp Ser Gln His Arg
   145                 150                 155                 160
   Ser His Arg Gln Met Val Thr Thr Thr Asn Pro Leu Ile Arg His Glu
                   165                 170                 175
   Asn Arg Met Val Leu Ala Ser Thr Thr Ala Lys Ala Met Glu Gln Met
               180                 185                 190
   Ala Gly Ser Ser Glu Gln Ala Ala Glu Ala Met Glu Val Ala Ser Gln
           195                 200                 205
   Ala Arg Gln Met Val Gln Ala Met Arg Thr Ile Gly Thr His Pro Ser
       210                 215                 220
   Ser Ser Ala Gly Leu Lys Asn Asp Leu Leu Glu Asn Leu Gln Ala Tyr
   225                 230                 235                 240
   Gln Lys Arg Met Gly Val Gln Met Gln Arg Phe Lys
                   245                 250
<210> 3
   <211> 9
   <212> PRT
   <213> Influenza virus
<400> 3
   Gly Ile Leu Gly Phe Val Phe Thr Leu
   1               5
<210> 4
   <211> 10
   <212> PRT
   <213> Influenza virus
<400> 4
   Ile Leu Gly Phe Val Phe Thr Leu Thr Val
   1               5                   10
<210> 5
   <211> 9
   <212> PRT
   <213> Influenza virus
<400> 5
   Ser Ile Ile Pro Ser Gly Pro Leu Lys
   1               5
<210> 6
   <211> 8
   <212> PRT
   <213> Influenza virus
<400> 6
   Met Gly Leu Ile Tyr Asn Arg Met
   1               5
<210> 7
   <211> 8
   <212> PRT
   <213> Influenza virus
<400> 7
   Ala Ser Cys Met Gly Leu Ile Tyr
   1               5
<210> 8
   <211> 9
   <212> PRT
   <213> Influenza virus
<400> 8
   Ile Leu Ser Pro Leu Thr Lys Gly Ile
   1 5
<210> 9
   <211> 10
   <212> PRT
   <213> Influenza virus
<400> 9
   Ile Leu Ser Pro Leu Thr Lys Gly Ile Leu
   1 5 10
<210> 10
   <211> 12
   <212> PRT
   <213> Influenza virus
<400> 10
   Ala Tyr Gln Lys Arg Met Gly Val Gln Met Gln Arg
   1 5 10
<210> 11
   <211> 10
   <212> PRT
   <213> Influenza virus
<400> 11
   Leu Glu Asn Leu Gln Ala Tyr Gln Lys Arg
   1 5 10
<210> 12
   <211> 12
   <212> PRT
   <213> Influenza virus
<400> 12
   Gly Pro Leu Lys Ala Glu Ile Ala Gln Arg Leu Glu
   1 5 10
<210> 13
   <211> 9
   <212> PRT
   <213> Influenza virus
<400> 13
   Arg Lys Leu Lys Arg Glu Ile Thr Phe
   1               5
<210> 14
   <211> 10
   <212> PRT
   <213> Influenza virus
<400> 14
   Arg Lys Leu Lys Arg Glu Ile Thr Phe His
   1               5                   10
<210> 15
   <211> 10
   <212> PRT
   <213> Influenza virus
<400> 15
   Ser Leu Leu Thr Glu Val Glu Thr Tyr Val
   1               5                   10
<210> 16
   <211> 8
   <212> PRT
   <213> Influenza virus
<400> 16
   Ile Ile Pro Ser Gly Pro Leu Lys
   1               5
<210> 17
   <211> 8
   <212> PRT
   <213> Influenza virus
<400> 17
   Leu Glu Asp Val Phe Ala Gly Lys
   1               5
<210> 18
   <211> 12
   <212> PRT
   <213> Influenza virus
<400> 18
   Glu Ile Ser Leu Ser Tyr Ser Ala Gly Ala Leu Ala
   1               5                   10
<210> 19
   <211> 50
   <212> PRT
   <213> Influenza virus
<400> 19
   Glu Ile Ser Leu Ser Tyr Ser Ala Gly Ala Leu Ala Ser Cys Met Gly
   1 5 10 15
   Leu Ile Tyr Asn Arg Met Gly Ala Val Thr Thr Glu Val Ala Phe Gly
   20 25 30
   Leu Val Cys Ala Thr Cys Glu Gln Ile Ala Asp Ser Gln His Arg Ser
   35 40 45
   His Arg
   50
<210> 20
   <211> 47
   <212> PRT
   <213> Influenza virus
<400> 20
   Glu Ile Ser Leu Ser Tyr Ser Ala Gly Ala Leu Ala Ser Cys Met Gly
   1 5 10 15
   Leu Ile Tyr Asn Arg Met Gly Ala Val Thr Thr Glu Val Ala Phe Gly
   20 25 30
   Leu Val Cys Ala Thr Cys Glu Gln Ile Ala Asp Ser Gln His Arg
   35 40 45
<210> 21
   <211> 9
   <212> PRT
   <213> Influenza virus
<400> 21
   Leu Ser Tyr Ser Thr Gly Ala Leu Ala
   1 5
<210> 22
   <211> 9
   <212> PRT
   <213> Influenza virus
<400> 22
   Leu Ser Tyr Ser Ala Gly Ala Leu Ala
   1               5
<210> 23
   <211> 9
   <212> PRT
   <213> Influenza virus
<400> 23
   Leu Asn Tyr Ser Thr Gly Ala Leu Ala
   1               5
<210> 24
   <211> 9
   <212> PRT
   <213> Influenza virus
<400> 24
   Leu Gly Tyr Ser Thr Gly Ala Leu Ala
   1               5
<210> 25
   <211> 9
   <212> PRT
   <213> Influenza virus
<400> 25
   Leu Ser Tyr Ser Thr Gly Ala Leu Thr
   1               5
<210> 26
   <211> 9
   <212> PRT
   <213> Influenza virus
<400> 26
   Phe Ser Tyr Ser Ala Gly Ala Leu Ala
   1               5
<210> 27
   <211> 6
   <212> PRT
   <213> Influenza virus
<220>
   <221> misc_feature
   <222> 3
   <223> Xaa is Ala or Thr
<400> 27
   Tyr Ser Xaa Gly Ala Leu
   1 5
<210> 28
   <211> 75
   <212> PRT
   <213> Influenza virus
<400> 28
   Ser Leu Leu Thr Glu Val Glu Thr Tyr Val Leu Ser Ile Ile Pro Ser
   1 5 10 15
   Gly Pro Leu Lys Ala Glu Ile Ala Gln Arg Leu Glu Asp Val Phe Ala
   20 25 30
   Gly Lys Asn Thr Asp Leu Glu Val Leu Met Glu Trp Leu Lys Thr Arg
   35 40 45
   Pro Ile Leu Ser Pro Leu Thr Lys Gly Ile Leu Gly Phe Val Phe Thr
   50 55 60
   Leu Thr Val Pro Ser Glu Arg Gly Leu Gln Arg
   65 70 75
<210> 29
   <211> 76
   <212> PRT
   <213> Influenza virus
<400> 29
   Ser Leu Leu Thr Glu Val Glu Thr Tyr Val Leu Ser Ile Ile Pro Ser
   1 5 10 15
   Gly Pro Leu Lys Ala Glu Ile Ala Gln Arg Leu Glu Asp Val Phe Ala
   20 25 30
   Gly Lys Asn Thr Asp Leu Glu Val Leu Met Glu Trp Leu Lys Thr Arg
   35 40 45
   Pro Ile Leu Ser Pro Leu Thr Lys Gly Ile Leu Gly Phe Val Phe Thr
   50 55 60
   Leu Thr Val Pro Ser Glu Arg Gly Leu Gln Arg Arg
   65 70 75
<210> 30
   <211> 12
   <212> PRT
   <213> Influenza virus
<400> 30
   Ser Leu Leu Thr Glu Val Glu Thr Tyr Val Leu Ser
   1 5 10